# EUROPÄISCHE PATENTSCHRIFT

(11) **EP 3 818 097 B1**
(45) Veröffentlichungstag und Bekanntmachung des Hinweises auf die Patenterteilung: **17.08.2022**
(21) Anmeldenummer: 19732043.5
(22) Anmeldetag: 24.06.2019
(51) Int. Cl.: C08G 71/04, C07D 327/04, C09J 175/04

(54) **KLEBEVERFAHREN UNTER VERWENDUNG VON 2-KOMPONENTENKLEBSTOFFEN AUF BASIS VON VERBINDUNGEN MIT CYCLOTHIOCARBONAT-EINHEITEN**
ADHESIVE METHOD USING TWO-COMPONENT ADHESIVES BASED ON COMPOUNDS HAVING CYCLOTHIOCARBONATE UNITS
PROCÉDÉ DE COLLAGE METTANT EN OEUVRE DES ADDITIFS À 2 CONSTITUANTS BASÉS SUR DES COMPOSÉS COMPRENANT DES MOTIFS DE CYCLOTHIOCARBONATE

(30) Priorität: 04.07.2018 EP 18181676
(43) Veröffentlichungstag der Anmeldung: 12.05.2021
(73) Patentinhaber: BASF SE, 67056 Ludwigshafen am Rhein (DE)
(72) Erfinder: LICHT, Ulrike, 67056 Ludwigshafen (DE); RUDOLF, Peter, 67056 Ludwigshafen (DE); JEGELKA, Markus, 67056 Ludwigshafen (DE); THIEL, Indre, 67056 Ludwigshafen (DE); SCHUMACHER, Karl-Heinz, 67056 Ludwigshafen (DE)
(74) Vertreter: BASF IP Association
(86) Internationale Anmeldenummer: PCT/EP2019/066687
(87) Internationale Veröffentlichungsnummer: WO 2020/007634

(56) Entgegenhaltungen:
- EP-A1- 0 943 660
- WO-A2-2012/085120
- US-A1- 2016 206 520
- M. LUO ET AL: "Synthesis of cyclic monothiocarbonates via the coupling reaction of carbonyl sulfide (COS) with epoxides", CATALYSIS SCIENCE & TECHNOLOGY, Bd. 6, Nr. 1, 1. Januar 2016 (2016-01-01), Seiten 188-192, XP055518365, United Kingdom ISSN: 2044-4753, DOI: 10.1039/C5CY00977D

## Beschreibung

Beschrieben wird ein Klebeverfahren unter Verwendung eines 2-Komponentenklebstoffs auf Basis einer Verbindung A mit Cyclothiocarbonat-Einheit und einer Härterverbindung B mit primären oder sekundären Amingruppen.

Als Klebstoffe werden häufig zweikomponentige Systeme von Polyurethanen auf Basis von Polyisocyanaten verwendet, in denen Isocyanatkomponenten mit Polyolkomponenten zu einem hochmolekularen Polyurethanpolymer reagieren. Diese Systeme werden entweder als lösemittelfreie und wasserfreie reaktive Einhundertprozentsysteme appliziert oder als in einem organischen Lösemittel gelöster Klebstoff. Die Beschichtungsstoffe werden mittels eines geeigneten Auftragssystems auf ein erstes Substrat aufgetragen und dann wird ggf. nach Verdampfen des Lösemittels ausgehärtet. Vorteilhaft sind die sich ergebenden hohen Verbundfestigkeiten in Kombinationen unterschiedlichster Folienmaterialien.

Die in herkömmlichen Zweikomponentenklebstoffen enthaltenen reaktiven, monomeren niedermolekularen (Poly)isocyanatverbindungen stellen ein toxikologisches Risiko dar, insbesondere wenn diese leichtflüchtig sind oder migrieren können. Das betrifft zum einen die Verarbeitung dieser Klebstoffe bei deren Anwendung, weil die Isocyanate in der Regel eine hohe Toxizität und ein hohes allergenes Potential aufweisen. Zum anderen besteht die Gefahr, dass bei flexiblen Substraten nicht vollständig abreagiertes aromatisches Isocyanat durch das Substrat migriert und dort durch Wasseranteile zu carzinogenen aromatischen Aminen hydrolysiert. Gewünscht sind daher Isocyanat-freie Zweikomponentensysteme für härtbare Klebstoffzusammensetzungen mit möglichst guten Klebewerten und mit guten Härtungseigenschaften möglichst bereits bei Raumtemperatur und ohne Erwärmung, d.h. kalthärtend.

Alternativen zu Polyurethane bildenden Isocyanat/Alkohol-Systemen sind bekannt. So reagieren cyclische Carbonate mit Aminen zu Urethanen und werden in der Literatur auch als "NISO" oder "NIPU" bezeichnet. 2K-Systeme aus Aminen und unsubstituierten, cyclischen Carbonaten sind nicht bei Raumtemperatur härtend sondern benötigen zur Härtung Erwärmen auf Temperaturen von bis zu 80°C . Deshalb wurden schon andere Wege zu schnellerem Umsatz von cyclischen Carbonaten mit Aminen beschritten. Die WO 2018/054713 und die WO 2016/202652 beschreiben die Verwendung von Exovinylen-cyclocarbonaten in Klebstoffen. Die WO 2017/207461 beschreibt die Verwendung von Cyclocarbonatamiden in Klebstoffen.

Cyclocarbonate mit Schwefelatomen im Ring sind bekannt als schneller härtend, aber die Synthese erfordert stark giftige und teure Einsatzstoffe wie CS2. Die WO 2006/005386 beschreibt härtbare Zusammensetzungen auf Basis von u.a. cyclischen Thiocarbonaten und Aminen, bei denen der Schwefel im cyclischen Thiocarbonat als C=S-Gruppe gebunden vorliegt. Die WO 2016/185106 beschreibt Kohlenwasserstoffpolymere mit zwei cyclischen Dithiocarbonatendgruppen, wobei die cyclischen Dithiocarbonate die Gruppe-S-C(=S)-O- enthalten. Die WO 2012/085120 beschreibt die Verwendung von Thiocarbonaten in härtbaren Epoxidharzformulierungen.

Die Herstellung von cyclischen Monothiocarbonaten wird beschrieben in EP17186542.1 und in EP17186545.4. Ausgehärtete Polyurethane, die gebildet sind aus cyclischen Monothiocarbonaten und Diaminen, werden beschrieben in EP17186543.9 und in EP17186544.7.

Die der vorliegenden Erfindung zu Grunde liegende Aufgabe war es, eine Alternative für auf der Reaktion mit Isocyanaten beruhende, Polyurethan bildende 2-Komponenten-Klebstoffe zur Verfügung zu stellen. Die Ausgangsstoffe sollten möglichst unbedenklich, gut zugänglich und mit Aminen hochreaktiv sein, und möglichst bereits bei Raumtemperatur ohne Erwärmen aushärten und möglichst gute Klebewerten ergeben, z.B. bei Anwendungen als Laminierklebstoff oder als Strukturklebstoff.

Es wurde gefunden, dass sowohl die unten näher beschriebenen monofunktionellen cyclischen Monothiocarbonate als auch multifunktionelle cyclische Monothiocarbonate (d.h. Verbindungen mit mehr als einer cyclischen Monothiocarbonatgruppe) mit Aminen bei Raumtemperatur in 24 h oder weniger zu Urethanthiolen reagieren, die in einer weiteren Folgereaktion, z.B. mit Doppelbindungen oder mit Epoxiden zu Polythioethern oder durch Disulfidbildung weiter härten können und sich so zweikomponentige Klebstoffe formulieren lassen, die einerseits eine günstige offene Zeit von Minuten bis Stunden aufweisen, andererseits aber innerhalb von 12 h bei Raumtemperatur bereits zu belastbaren Verklebungen führen.

Gegenstand der vorliegenden Erfindung ist ein Klebeverfahren, wobei zwei Substrate miteinander verklebt werden, indem auf die Oberfläche mindestens eines der Substrate ein härtbarer, noch nicht ausgehärteter, vorzugsweise bei Raumtemperatur fluider oder flüssiger 2-Komponentenklebstoff aufgetragen wird, welcher
(a) in einer ersten Komponente mindestens eine Verbindung A enthält mit mindestens einer Cyclothiocarbonat-Einheit mit Fünfringstruktur, wobei drei Glieder des Fünfrings die Struktur -O-C(=O)-S- aufweisen, die beiden übrigen Glieder des Fünfrings C-Atome sind; und
(b) in einer zweiten Komponente mindestens eine Härterverbindung B enthält, welche ausgewählt ist aus Verbindungen, welche mindestens eine funktionelle Gruppe aufweisen, die ausgewählt ist aus primären Amingruppen und sekundären Amingruppen, wobei die mindestens eine funktionelle Gruppe auch in maskierter, latent reaktiver Form vorliegen kann.
In einem weiteren Schritt wird das mit dem Klebstoff beschichtete Substrat mit einem zweiten Substrat in Kontakt gebracht und der Klebstoff wird ausgehärtet.

Optional kann in der ersten Komponente, in der zweiten Komponente und/oder in einer weiteren Komponente des Klebstoffs mindestens eine Verbindung C enthalten sein, welche mindestens eine mit SH-Gruppen reaktive funktionelle Gruppe aufweist. Vorzugsweise ist die mit SH-Gruppen reaktive funktionelle Gruppe ausgewählt aus Gruppen mit mindestens einer ethylenisch ungesättigten Bindung und Epoxygruppen, wobei im Falle von Verbindungen mit Epoxygruppen diese in der ersten und/oder in der weiteren (dritten) Komponente des Klebstoffs enthalten sind. Verbindung A enthält eine oder mehrere cyclische Monothiocarbonatgruppen, nachfolgend abkürzend auch als Monothiocarbonat bezeichnet.

Vorzugsweise sind mindestens zwei der Verbindungen A, B und C multifunktionell. Eine multifunktionelle Verbindung A weist zwei oder mehr der cyclischen Monothiocarbonatgruppen auf. Eine multifunktionelle Verbindung B weist entweder zwei oder mehr funktionellen Gruppen auf, die ausgewählt sind aus primären Amingruppen und sekundären Amingruppen oder Verbindung B weist mindestens eine funktionelle Gruppe auf, ausgewählt aus primären Amingruppen und sekundären Amingruppen und mindestens eine mit SH-Gruppen reaktive Gruppe. Eine multifunktionelle Verbindung C weist zwei oder mehr der mit SH-Gruppen reaktiven Gruppen auf.

Der Begriff "härtbar, noch nicht ausgehärtet" bedeutet, dass sowohl Cyclothiocarbonatgruppen der Verbindung A als auch damit reaktive funktionelle Gruppen der Verbindung B vorliegen, die nocht nicht miteinander reagiert haben. Härtung zeigt sich z.B. durch eine Zunahme der Nullviskosität. Die Nullviskosität der gemischten Komponenten bei 23°C vor dem Beschichten der Substrate ist vorzugsweise kleiner 3000 Pa s, oder kleiner 1500 Pa s, besonders bevorzugt kleiner 300 Pa s. Vorzugsweise ist die Nullviskosität der gemischten Komponenten bei 70°C vor dem Beschichten der Substrate kleiner 300 Pa s.

Molekulargewichte von Polymeren beziehen sich hierin auf das gewichtsmittlere Molekulargewicht, gemessen durch Gelpermeationschromtatographie in THF mit Polystyrolstandard, sofern nichts anderes angegeben ist.

### Bevorzugt ist ein Klebeverfahren, wobei

(a) Verbindung A eine Anzahl von n_{A} der Cyclothiocarbonat-Einheiten aufweist, wobei n_{A} eine ganze Zahl größer oder gleich 1, vorzugsweise 2 bis 1000 ist;
(b) Verbindung B eine Anzahl von n_{B} der funktionellen Gruppen und eine Anzahl von n_{C2} an mit SH-Gruppen reaktiven ethylenisch ungesättigten Doppelbindungen aufweist, wobei n_{B} eine ganze Zahl größer oder gleich 1, vorzugsweise 2 bis 1000 ist und n_{C2} eine ganze Zahl größer oder gleich 0 ist; und
(c) optional in der ersten Komponente, in der zweiten Komponente und/oder in einer weiteren Komponente mindestens eine Verbindung C enthalten ist, welche eine Anzahl n_{C3} an mit SH-Gruppen reaktiven funktionellen Gruppen enthält und n_{C3} eine ganze Zahl größer oder gleich 1, vorzugsweise 2 bis 1000 ist;
   mit der Massgabe, dass mindestens eine der Zahlen n_{A} und der Summe aus n_{B} + n_{C2} größer oder gleich 2 ist;
   und dass wenn n_{C2} Null ist, dann entweder n_{C3} größer oder gleich 2 ist oder sowohl n_{A} als auch n_{B} größer oder gleich 2 sind oder sowohl n_{A} als auch n_{B} als auch n_{C3} größer oder gleich 2 sind.

Das Monothiocarbonat kann weitere Heteroatome enthalten, z.B. Sauerstoff, Schwefel, Stickstoff, Chlor oder Silicium, beispielsweise in Form von funktionellen Gruppen, ausgewählt aus Epoxygruppen, Ethergruppen, Hydroxygruppen, Ketogruppen, Aldehydgruppen, Estergruppen, Carboxygruppen, Thioethergruppen, Thiolgruppen, tertiären Amingruppen. Vorzugsweise hat das Monothiocarbonat maximal eine funktionelle Gruppe außer der Monothiocarbonatgruppe.

Verbindung A kann ein Monomer, ein Oligomer oder ein Polymer sein. Verbindung A kann beispielsweise bis zu 1000, oder bis zu 500 oder bis zu 100 fünfgliedrige cyclische Monothiocarbonatgruppen aufweisen. Vorzugsweise hat Verbindung A 1 bis 10 oder 1 bis 5, besonders bevorzugt 1, 2 oder 3, insbesondere 1 oder 2 fünfgliedrige cyclische Monothiocarbonatgruppen.

Verbindung A kann ein Molekulargewicht haben von z.B. 104 bis zu 500.000 oder bis zu 100.000 g/mol, z.B. von 104 bis 1000 g/mol oder von 104 bis 500 g/mol. Im Fall von Polymeren handelt es sich dabei um das gewichtsmittlere Molekulargewicht, gemessen durch Gelpermeationschromtatographie gegen Polystyrol als Standard. Vorzugsweise ist das Molekulargewicht größer als 1000 g/mol.

Vorzugsweise hat Verbindung A keine primären oder sekundären Aminogruppen. Vorzugsweise hat Verbindung A keine anderen funktionellen Gruppen außer Monothiocarbonatgruppen, Carbonsäureestergruppen und Ethergruppen.

Bevorzugte Verbindungen A sind beispielsweise solche der Formel (I) 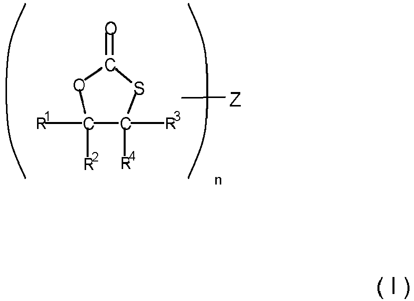 wobei R¹ bis R⁴ unabhängig voneinander Wasserstoff oder eine organische Gruppe mit vorzugsweise bis zu 50 C-Atomen bedeuten, wobei alternativ R², R⁴ und die beiden C-Atome der Monothiocarbonatgruppe zusammen einen fünf- bis zehngliedrigen Ring bilden können, und wobei eine der Gruppen R¹ bis R⁴ eine Verbindungsgruppe zu Z ist, wobei die Verbindungsgruppe auch eine chemische Bindung sein kann, n eine ganze Zahl von größer oder gleich 1 bedeutet und Z für Wasserstoff oder für eine n-valente organische Gruppe steht.

In den Fällen, in denen R¹ bis R⁴ organischen Gruppen bedeuten, handelt es sich vorzugsweise um organische Gruppen mit bis zu 30, besonders bevorzugt bis zu 20 C-Atomen. Vorzugweise bilden R² und R⁴ keine fünf- bis zehngliedrigen Ringe mit den zwei C-Atomen der Monothiocarbonatgruppe.

In den Fällen, in denen R¹ bis R⁴ organischen Gruppen bedeuten, können diese die oben genannten Heteroatome und funktionellen Gruppen aufweisen, z.B. Sauerstoff, Stickstoff, Schwefel, Silicium oder Chlor, vorzugsweise Sauerstoff oder Chlor. R¹ bis R⁴ können z.B. Sauerstoff enthalten in Form von Ether-, Hydroxy-, Aldehyd-, Keto- oder Carboxygruppen. Vorzugsweise ist die organische Gruppe eine aliphatische organische Gruppe mit bis zu 30 C-Atomen, die Sauerstoff, Stickstoff oder Chlor enthalten kann.

Bevorzugt ist die organische Gruppe ausgewählt aus Alkyl, -CH₂-O-R⁵ , -CH₂-O-C(=O)-R⁶ oder -CH₂-NR⁷R⁸ wobei R⁵ bis R⁸ organische Gruppen mit bis zu 30, vorzugsweise bis zu 20 C-Atomen sind. Insbesondere stehen R⁵ bis R⁸ für aliphatische oder aromatische Gruppen, die Sauerstoff enthalten können, z.B. in Form von Ethergruppen. Vorzugsweise stehen R⁵ bis R⁸ für aliphatische Kohlenwasserstoffgruppen, z.B. Alkylgruppen mit 1 bis 10 C-Atomen, Alkoxygruppen oder Polyalkoxygruppen. Besonders bevorzugt sind R⁵ bis R⁸ aliphatische Kohlenwasserstoffgruppen, insbesondere Alkylgruppen mit 1 bis 10 C-Atomen. Besonders bevorzugt ist die organische Gruppe -CH₂-O-R⁵ oder-CH₂-O-C(=O)-R⁶. Vorzugsweise sind ein bis drei, besonders bevorzugt zwei oder drei der Substituenten R¹ bis R⁴ in Formel (I) Wasserstoff und die übrigen der Substituenten R¹ bis R⁴ organische Gruppen bzw. die Verbindungsgruppe zu Z. Besonders bevorzugt sind drei der Substituenten R¹ bis R⁴ in Formel (I) Wasserstoff und R¹ oder R² ist eine organische Gruppe bzw. die Verbindungsgruppe zu Z.

Eine der Gruppen R¹ bis R⁴ ist die Verbindungsgruppe zu Z. Vorzugsweise ist die Verbindungsgruppe lediglich eine Bindung oder -CH₂-O- oder -CH₂-O-C(=O)- oder -CH₂-NR²⁰- wobei R²⁰ eine aliphatische Gruppe bedeutet, vorzugsweise eine Alkylgruppe mit bis zu 20 C-Atomen. Vorzugsweise ist die Verbindungsgruppe lediglich eine Bindung oder -CH₂-O- oder -CH₂-O-C(=O)-. Besonders bevorzugt ist die Verbindungsgruppe -CH₂-O-.

In Formel (I) ist n gleich 1 oder eine Zahl größer oder gleich 2, z.B. von 1 bis 1000, von 2 bis 100 oder von 2 bis 10. Besonders bevorzugt ist n gleich 1 oder von 2 bis 5, insbesondere 2 oder 3. Am meisten bevorzugt ist n gleich 2.

Z ist Wasserstoff oder eine n-valente organische Gruppe. Im Fall von hohen Werten für n, z.B. 10 bis 1000 oder mehr, kann Z eine polymere Gruppe sein, insbesondere eine Polymerkette, hergestellt durch Polymerisation oder Copolymerisation, z.B. durch radikalische Polymerisation von ethylenisch ungesättigten Monomeren, durch Polykondensation oder durch Polyaddition. Beispielsweise werden Polyester oder Polyamide erhalten durch Polykondensation unter Abspaltung von Wasser oder Alkohol oder Polyurethane oder Polyharnstoffe werden erhalten durch Polyaddition. Derartige polymere Verbindungen der Formel (I) sind z.B. Polymere erhältlich durch radikalische Polymerisation oder Copolymerisation von ethylenisch ungesättigten Monomeren enthaltend Epoxygruppen, die anschließend in Monothiocarbonatgruppen überführt werden.

Vorzugsweise ist Z eine n-valente organische Gruppe mit bis zu 50 C-Atomen, insbesondere bis zu 30 C-Atomen, und die weitere Elemente enthalten kann außer Kohlenstoff und Wasserstoff, wobei n eine Zahl ist von 2 bis 5, vorzugsweise 2 oder 3, besonders bevorzugt 2. Besonders bevorzugt enthält die n-valente organische Gruppe nur Kohlenstoff und Wasserstoff und optional Sauerstoff und ansonsten keine weiteren Elemente.

Eine bevorzugte Gruppe Z ist auch eine Polyalkoxylengruppe der Formel G1:

(V-O-)ₘV

wobei V eine C2- bis C20 Alkylengruppe bedeutet und m eine Zahl von größer oder gleich 1 ist. Vorzugsweise ist die C2- bis C20 Alkylengruppe eine C2- bis C4-Alkylengruppe, insbesondere Ethylen oder Propylen. Die Zahl m kann z.B. von 1 bis 100, insbesondere von 1 bis 50 sein.

Eine weitere bevorzugte Gruppe Z ist eine Gruppe der Formel G2: 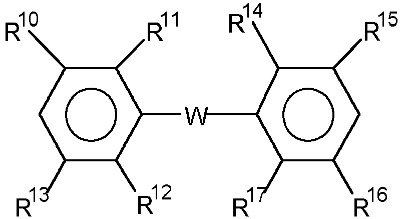 wobei W eine bi-valente organische Gruppe ist mit bis zu 10 C-Atomen und n gleich 2 ist und R¹⁰ bis R¹⁷ unabhängig voneinander H oder eine C1- bis C4 Alkylgruppe bedeuten. Vorzugsweise sind mindestens sechs, besonders bevorzugt sind alle der Substituenten R¹⁰ bis R¹⁷ Wasserstoff. Die Gruppen W sind z.B.:

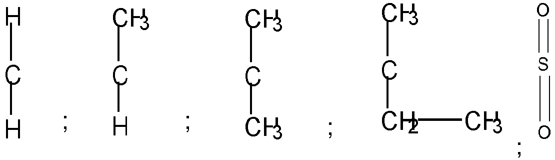

Vorzugsweise ist W eine organische Gruppe, die nur aus Kohlenstoff und Wasserstoff besteht. Besonders bevorzugt ist W 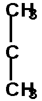 was der Struktur von Bisphenol A entspricht.

Bevorzugte Verbindungen A mit mindestens 2 fünfgliedrigen cyclischen Monothiocarbonaten sind Verbindungen, erhältlich durch Umwandeln aller Epoxygruppen der folgenden Epoxyverbindungen in fünfgliedrige cyclische Monothiocarbonatgruppen:
Nicht-glycidyl Epoxide:
   1,2:5,6-Diepoxyhexahydro-4,7-methanoindan, Bis-(3,4-Epoxycyclohexylmethyl)adipat, 1,4-Cyclohexandimethanol-bis(3,4-epoxycyclohexan)carboxylate, 1-Methyl-4-(2-methyloxiranyl)-7-oxab-icyclo[4.1.0]heptan, 4-Vinylcyclohexendioxid, 1,2,5,6-Diepoxycyclooctan, 1,2,7,8-Diepoxyoctan, Dicyclopentadiendioxid, epoxidierte pflanzliche Öle und deren Derivative, z.B. Sojabohnenöl oder dessen Derivate.
Glycidylether:
   Bisphenol A diglycidylether (BADGE), Hydriertes BADGE, Glycidylether von anderen Di-, Tri, Tetra- und Polyolen z.B. Butandiol-diglycidylether, Trimethylolpropan-triglycidylether, Pentaerythritoltetraglycidyl ether, Sorbitolpolyglycidylether, Isosorbiddiglycidylether, Methylphenlypropandioldiglycidylether, einschließlich oligomere und polymere Glycidylether, z.B. Polypropylenglycoldiglycidylether, Polyglycerolpolyglycidylether, Novolac-glycidylether, Oligomere oder Polymere erhätlich durch Reaktion von Bisphenol A mit einem Überschuss an Epichlorhydrin.
Glycidylester: Tetrahydrophthalsäurediglycidylester, Diglycidyl-1,2-cyclohexanedicarboxylat, Diglycidylorthophthalat.
Glycidyl amine:
   N,N-Diglycidyl-4-glycidyloxyanilin, Tetraglycidylmethylendianilin
Glycidylimide:
   Triglycidylisocyanurat

Bevorzugte Verbindungen A mit einer einzigen Monothiocarbonatgruppe sind:

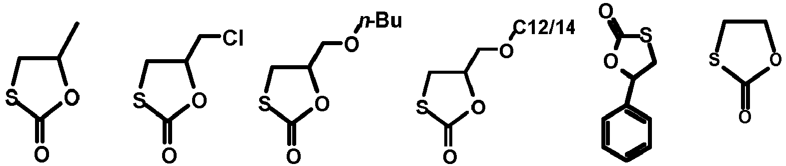

Bevorzugte Verbindungen A mit zwei oder mehr Monothiocarbonatgruppen sind:

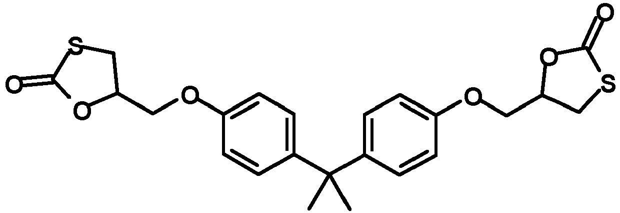

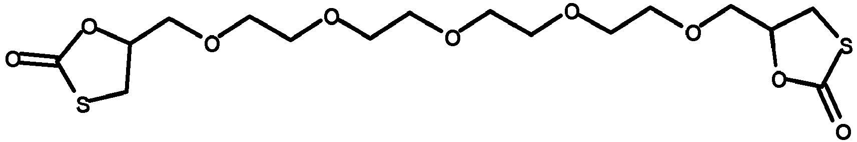

Bevorzugte Verbindungen A mit zwei oder mehr Monothiocarbonatgruppen sind auch von mehrfach epoxidierten Fetten und Fettsäurederivaten abgeleitete Monothiocarbonate.

Bevorzugte Verbindungen A sind solche der Formel II 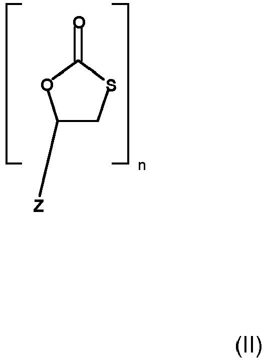 wobei n eine Zahl größer oder gleich 1, vorzugsweise größer oder gleich 2 ist, und Z für eine n-valente organische Gruppe oder für Wasserstoff steht, vorzugsweise für Alkyl, Aryl, Alkenyl, oder Aralkyl, mit vorzugsweise jeweils bis zu 50 C-Atomen, insbesondere bis zu 30 C-Atomen, wobei die Gruppe Z substituiert oder unsubstituiert sein kann und wobei die Gruppe Z unterbrochen sein kann durch O, Halogen, S, C=O, O-C=O, O-(C=O)-O oder (C=O)-NR, wobei R Wasserstoff oder eine organische, vorzugsweise aliphatische Gruppe bedeutet, insbesondere eine Alkylgruppe mit vorzugsweise bis zu 20 C-Atomen.

Bevorzugte Verbindungen A sind auch solche der Formel III 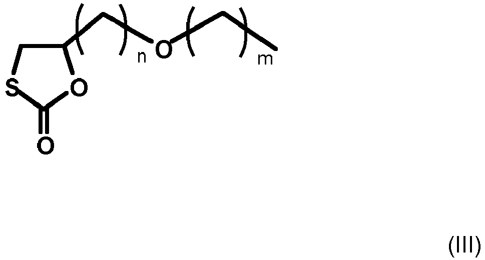 wobei n eine Zahl von 1 bis 10, vorzugsweise 1 ist und m eine Zahl von 0 bis 9, vorzugsweise1 bis 5 ist

Bevorzugte Verbindungen A sind auch solche der Formel (IV) 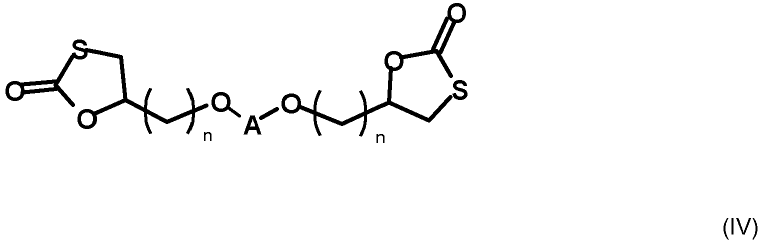 wobei n eine Zahl von 1 bis 10, vorzugsweise 1 ist und A ein Gruppe ist ausgewählt aus -Ph-CRₐR_{b}-Ph- und -(CH₂-CH₂-O)ₘ CH₂-CH₂- , wobei Rₐ und R_{b} unabhängig voneinander für H oder C1 bis C4-Alkyl, vorzugsweise für Methyl stehen; und m eine Zahl von 0 bis 10, vorzugsweise 1 bis 5 ist.

Bevorzugte Verbindungen A sind auch solche der Formel (IV), bei denen die Gruppe -A- eine Gruppe -(CH₂)ₚ- ist, wobei p für eine Zahl von 1 bis 10 steht.

Bevorzugte Verbindungen A sind auch solche der Formel (IV), bei denen die Gruppe -A- eine Gruppe -C(=O)-B-C(=O)- ist, wobei B für eine organische Gruppe, vorzugsweise für eine Kohlenwasserstoffgruppe steht, z.B. für -(CH₂-CH₂-O)ₘ-CH₂-CH₂- oder für -(CH₂)ₚ-, wobei m und p jeweils für eine Zahl von 1 bis 10 stehen.

Bevorzugt ist auch eine Verbindung, bei der die Gruppe -A- für die Gruppe 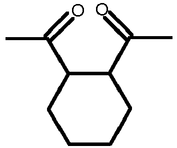 steht.

### Synthese der Verbindung A

Einige Syntheseverfahren für Monothiocarbonate sind in der Literatur beschrieben. Gemäß US 3072676 und US 3201416 können Ethylenemonothiocarbonate hergestellt werden durch ein zweistufiges Verfahren. In einem ersten Schritt reagieren Mercaptoethanol und Chlorcarboxylate zu Hydroxyethylthiocarbonaten, welches in einem zweiten Schritt erwärmt wird in Gegenwart eines Metallsalzkatalysators und das Ethylenmonothiocarbonat bildet. Gemäß

US 3517029 werden Alkylenmonothiocarbonate erhalten durch Reaktion von Mercaptoethanol und einem Carbonatdiester in Gegenwart eines katalytisch wirksamen Thoriumsalzes. Gemäß US 3349100 weden Alkylenmonothiocarbonate erhalten durch Reaktion eines Epoxids mit Carbonylsulfid. Eine Synthese aus Phosgen und Hydroxymercaptanen ist beschrieben in US 2828318. Synthesen von fünfgliedrigen cyclischen Monothiocarbonaten sind auch beschrieben in EP17186542.1, EP17186545.4, EP17186543.9 und in EP17186544.7.

Ein Verfahren zur Herstellung von Verbindung A, insbesondere von Verbindungen der Formel (I) ist ein Verfahren wobei
a) eine Verbindung mit ein, zwei oder mehr Epoxygruppen (nachfolgend auch Epoxyverbindung genannt) als Startmaterial eingesetzt wird,
b) die Verbindung mit Phosgen oder einem Alkylchloroformat zu einem Addukt umgesetzt wird und
c) das Addukt mit einer Verbindung umgesetzt wird welche anionischen Schwefel enthält unter Bildung einer Verbindung die ein, zwei oder mehr fünfgliedrige, cyclische Monothiocarbonatgruppen enthält.

In Schritt b) wird vorzugsweise Phosgen eingesetzt. Der Begriff Phosgen umfasst auch Phosgeneersatzstoffe, d.h. Verbindungen welche Phosgen freisetzen, z.B. Triphosgen. Nachfolgend ist die Reaktion des Schrittes b) exemplarisch dargestellt für eine spezifische Epoxyverbindung mit Substituent R und mit Phosgen als Reaktant.

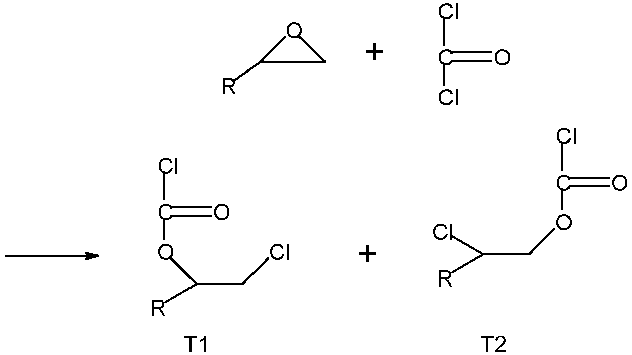

Es werden zwei Strukturisomere T1 und T2 von β-Chloroalkylchloroformat T1 and T2 erhalten. Vorzugsweise entsprechen mindestens 80 %, mindestens 90 %, oder mindestens 95% des Adduktes dem Isomer T1.

Die Reaktion der Epoxyverbindung mit Phosgen oder einem Alkylchloroformat kann in jedem stöchiometrischen Verhältnis erfolgen. Vorzugsweise wird ein hoher Überschuss an Epoxyverbindung vermieden. Vorzugsweise wird Phosgen bzw. Chloroformat in Mengen von 0.1 bis 5 mol, insbesondere 0.5 bis 2 mol pro mol Epoxygruppe eingesetzt. Besonders bevorzugt wird ein Überschuss an Phosgen bzw. Chloroformat eingesetzt. Mit mindestens äquimolaren Mengen von Phosgen bzw. Chloroformat werden nicht umgesetzte Epoxygruppen vermieden. Deshalb wird Phosgen bzw. Chloroformat vorzugsweise eingesetzt in Mengen von 0.9 bis 5 mol, 1 bis 2 mol, oder 1 bis 1.5 mol pro mol Epoxygruppe.

Phosgen und Chloroformat sind vorzugsweise Verbindungen der Formel 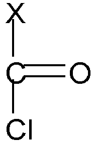 wobei X Cl ist im Falle von Phosgen oder -O-R5 im Fall von Chloroformat, wobei R5 C1- bis C4 Alkyl bedeutet.

Vorzugsweise erfolgt die Reaktion in Gegenwart eines Katalysators. Geeignete Katalysatoren sind Salze mit quaternären Ammoniumkation, z.B. Tetraalkylammoniumhalogenide, insbesondere die Chloride, z.B. Tetrabutylammoniumchlorid, Tetrahexylammoniumchlorid, Benzyltributylammoniumchlorid oder Trioctylmethylammoniumchlorid. Weitere Katalysatoren sind Hexaalkylguanidiniumhalogenide, insbesondere die Chloride, quarternäre Phosphoniumhalogenide, insbesondere die Chloride, Pyridine oder andere Verbindungen mit einem Stickstoff enthaltenden Ringsystem wie Imidazole oder alkylierte Imidazole. Bevorzugte Katalysatoren sind Salze mit quaternärem Ammoniumkation, insbesondere Salze von Tetraalkylammonium, z.B. Tetra(n-butyl)ammoniumchlorid. Vorzugsweise wird der Katalysator eingesetzt in Mengen von 0.001 bis 0.1 mol, insbesondere 0.005 bis 0.05 mol pro Mol Epoxygruppe.

Vorzugsweise wird Phosgen oder Alkylchloroformat zur Epoxyverbindung zugefügt. Da die Reaktion exotherm ist, erfolgt die Zugabe langsam und/oder unter Kühlung. Vorzugsweise wird die Reaktionstemperatur bei -40 bis 60°C, oder bei 5 bis 50°C gehalten.

Niedrigmolekulare Epoxyverbindugnen sind häufig flüssig, sodass ein zusätzliches Lösungsmittel nicht benötigt wird. Vorzugsweise wird ein Lösungsmittel verwendet, wenn die Epoxyverbindung bei 21°C fest ist. Geeignete Lösungsmittel sind zum Beispiel aprotische Lösungsmittel, z.B. Kohlenwasserstoffe einschließlich aromatische Kohlenwasserstoffe und chlorierte Kohlenwasserstoffe. Als Lösungsmittel für feste Epoxyverbindungen können auch flüssige Epoxyverbindungen verwendet werden.

Ausgehend von den wie oben beschrieben hergestellten β-Chloroalkylchlorformaten ist der der nachfolgende Reaktionsschritt exemplarisch mit Na₂S wie folgt:

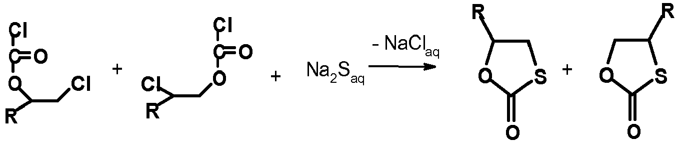

Dabei wird das Verhältnis der Strukturisomeren T1 und T2 in der Regel beibehalten.

In Schritt c) kann ein Lösungsmittel verwendet werden. Geeignete Lösungsmittel sind zum Beispiel aprotische Lösungsmittel, z.B. Kohlenwasserstoffe einschließlich aromatische Kohlenwasserstoffe und chlorierte Kohlenwasserstoffe oder hydrophile aprotische Lösungsmittel. z.B. Ether wie Tetrahydrofuran, Dioxan, Polyether (z.B. Glykoldiether, Glyme), Acetonitril oder Dimethylsulfoxid.

Das Produkt aus Schritt b) wird mit einer Verbindung mit anionischem Schwefel, vorzugsweise einem Salz umgesetzt. Der anionische Schwefel ist vorzugsweise S²⁻, ein Polysulfide (Sₚ)²⁻ wobei p eine Zahl von 2 bis 200, vorzugsweise 2 bis 10 ist, oder HS¹⁻. Das Kation des Salzes kann organisch oder anorganisch sein, vorzugsweise ein anorganisches Metallkation. Metallkationen sind z.B. Kationen von Alkali- oder Erdalkalimetallen, wie Natrium oder Kalium. Bevorzugte Schwefelsalze sind Na₂S, K₂S, NaSH und KSH oder deren Hydrate. Das Schwefelsalz kann in Kombination mit einer basischen Verbindung eingesetzt werden, insbesondere einem Metallhydroxid, z.B. NaOH oder KOH. Eine basische Verbindung wird vorzugsweise im Fall von SH⁻als Anion verwendet. Da die Reaktion exotherm ist, erfolgt die Zugabe des Salzes bzw. einer Lösung des Salzes langsam und/oder unter Kühlung. Vorzugsweise wird die Reaktionstemperatur bei -40 bis 60°C, oder bei -10 bis 50°C gehalten. Vorzugsweise wird das Salz in einer Menge von 0.5 bis 2.0 mol, besonders bevorzugt von 1.0 bis 2.0 mol oder von 1.0 bis 1.2 mol pro mol β-Chloroalkylchlorformatgruppe eingesetzt. Der Reaktionsschritt c) kann in Gegenwart eines Katalysators erfolgen, z.B. einem Phasentransferkatalysator wie Ammoniumsalze, heterocyclische Ammoniumsalze oder Phosphoniumsalze. Durch die Reaktion mit dem Schwefelsalz werden die β-Chloroalkylchlorformatgruppen in fünfgliedrige cyclische Monothiocarbonat-gruppen überführt. Dabei wird der fünfgliedrige Ring gebildet aus drei C-Atomen, einem O-Atom und einem S-Atom, wobei ein weiteres O-Atom über eine Doppelbindung mit dem C-Atom zwischen dem O-Atom und dem S-Atom des Ringsystems verbunden ist.

Der Zweikomponenten-Klebstoff enthält in einer zweiten Komponente mindestens eine Härterverbindung B, welche ausgewählt ist aus Verbindungen, welche mindestens eine funktionelle Gruppe aufweisen, die ausgewählt ist aus primären Amingruppen und sekundären Amingruppen (im Folgenden zusammen auch Aminhärter genannt), wobei die mindestens eine funktionelle Gruppe auch in maskierter, latent reaktiver Form vorliegen kann.

Vorzugsweise ist Verbindung B ausgewählt aus Polyaminen mit mindestens zwei primären oder sekundären Amingruppen. Besonders bevorzugt ist Verbindung B eine Verbindung ist mit mindestens zwei primären Aminogruppen. Wenn die Verbindung A zwei oder mehr Cyclothiocarbonatgruppen aufweist, können auch Härterverbindungen B eingesetzt werden, die nur eine einzige mit den Cyclothiocarbonatgruppen reaktive funktionelle Gruppe aufweisen, d.h. nur eine einzige primäre oder sekundäre Amingruppe.

Geeignete Monoamine sind z.B. Monoalkylamine und Dialkylamine mit vorzugsweise 1 bis 30 oder 1 bis 20 C-Atomen.

Bei dem erfindungsgemäßen Verfahren reagieren die Monothiocarbonate mit Aminhärtern zu Mercaptourethanen bzw. Mercaptopolyurethanen. Es entstehen hierbei Verbindungen mit einer oder mehreren Urethangruppen und einer oder mehrerer SH-Gruppen. Als Amine kommen hierbei primäre und sekundäre Amine mit Alkylgruppen, Arylgruppen, Aralkylgruppen sowie Alkarylgruppen als Reste in Frage. Primäre Amine reagieren schneller als sekundäre Amine; aliphatische Amine reagieren schneller als aromatische Amine. Insbesondere höhermolekulare Polyamine wie z.B. Jeffamine^{®} der Huntsman Corp. und Polyetheramine der BASF SE kommen hier in Frage.

Im Falle von primären Aminen mit der Formel R'-NH₂ kann die Umsetzung wie folgt dargestellt werden:

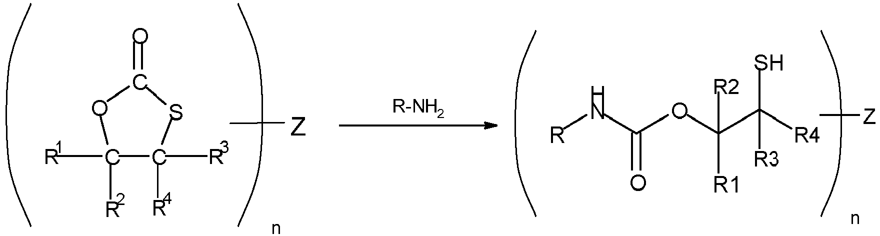

Abmischungen der Verbindungen A mit cyclischen Monothiocarbonatgruppen mit einer geeigneten, vorzugsweise mehrfunktionellen Härterverbindung B können als Zwei-Komponentenklebstoff eingesetzt werden mit der Verbindung A in einer ersten Komponente und der Härterverbindung B in einer zweiten Komponente. Verbindung A weist vorzgusweise mindestens 2 oder mehr cyclische Monothiocarbonatgruppen auf. Die Härterkomponente B weist vorzugsweise mindestens zwei funktionelle Gruppen auf, ausgewählt aus der Gruppe bestehend aus primären Aminogruppen und sekundären Aminogruppen. Bevorzugt ist auch eine Mischung aus mindestens einer Verbindung A mit 2 oder mehr cyclischen Monothiocarbonat-gruppen, mindestens einer monofunktionellen Härterverbindung B mit nur einer primären oder sekundären Amingruppe und einer Verbindung C mit zwei, drei oder mehr mit SH-Gruppen reaktiven funktionellen Gruppen, vorzugsweise Gruppen mit ethylenisch ungesättigten Bindungen oder Epoxygruppen.

Es können auch Mischungen unterschiedlicher Thiocarbonate eingesetzt werden, z.B. eine geringe Menge eines "schnell" reagierenden Thiocarbonats das Sofortfestigkeit aufbaut, ohne die Topfzeit des Klebers zu sehr zu reduzieren, plus ein langsames Thiocarbonat für die endgültige Aushärtung.

Vorzugsweise enthält der Zwei-Komponentenklebstoff mindestens einen Katalysator für die Katalysierung der Reaktion der cyclischen Monothiocarbonatgruppen mit den funktionellen Gruppen des Härters und/oder mindestens einen Katalysator für die Katalysierung der Reaktion des intermediär gebildeten Thiols mit SH-reaktiven Gruppen.

Es können auch Mischungen unterschiedlicher Härterverbindungen eingesetzt werden, z.B. eine geringe Menge eines schnellen Härters, der Sofortfestigkeit aufbaut, ohne die Topfzeit des Klebers zu sehr zu reduzieren, plus ein langsamer Härter für die endgültige Aushärtung.

Vorzugsweise sind die funktionellen Gruppen des Härters ausgewählt aus aliphatischen primären Aminogruppen und aliphatischen sekundären Aminogruppen.

Unter einem Zweikomponentenklebstoff versteht man einen Klebstoff, der vorzugsweise wenigstens zwei polyfunktionelle Klebstoffbestandteile enthält, die miteinander unter Bindungsbildung reagieren und dabei ein polymeres Netzwerk ausbilden. Dementsprechend enthalten Zweikomponentenklebstoffzusammensetzungen vorzugsweise neben wenigstens einer mehrfunktionellen Monothiocarbonatverbindung A zusätzlich wenigstens eine Verbindung B, die wenigstens 2 funktionelle Gruppen F, z.B. 2, 3, 4, 5, 6, 7, 8, 9 oder 10 funktionelle Gruppen F, aufweist, welche vorzugsweise unter aliphatischen primären Aminogruppen und sekundären Aminogruppen ausgewählt sind oder mindestens eine Verbindung C mit mindestens zwei mit SH-Gruppen reaktiven funktionellen Gruppen. Die Verbindungen B werden im Folgenden auch als Härter bezeichnet. Vorzugsweise wird die Menge an Härter so gewählt, dass das Molverhältnis von funktionellen Monothiocarbonat-Gruppen zu den funktionellen Gruppen F im Härter im Bereich von 1 : 10 bis 10:1, insbesondere im Bereich von 5 : 1 bis 1 : 5 und speziell im Bereich von 1 : 2 bis 2 : 1 liegt.

Der Härter kann eine niedermolekulare Substanz sein, d. h., sein Molekulargewicht liegt unterhalb 500 g/mol, oder eine oligomere oder polymere Substanz, die ein zahlenmittleres Molekulargewicht oberhalb 500 g/mol aufweist.

Für besonders gute Klebewirkungen ist es bevorzugt, dass entweder die Verbindung A oder die Härterverbindung B, oder Verbindung C, oder Verbindung A und Verbindung B oder Verbindung A und Verbindung C oder Verbindung B und Verbindung C oder Verbindungen A, B und C, jeweils mindestens eine flexible Spacergruppe enthalten. Eine flexible Spacergruppe ist eine lineare oder verzweigte Verbindungsgruppe, die ein Molekulargewicht von mindestens 200 g/mol aufweist. Die Spacergruppe kann die Gruppe R¹ oder einen Teil der Gruppe R¹ bzw. die Verbindungsgruppe zu Z oder einen Teil der Verbindungsgruppe zu Z in Formel (I) bilden und/oder die Spacergruppe kann sich zwischen zwei funktionellen Gruppen des Härters B befinden. Die Spacergruppe ist vorzugsweise ausgewählt aus Alkylengruppen, Polyethergruppen, Polycarbonatgruppen, Polyestergruppen und Poly(meth)acrylatgruppen. Spacergruppen sind z.B. lineare oder verzweigte Alkylengruppen mit mindestens 15 C-Atomen; Polyethergruppen der allgemeinen Formel -(A-O)ₘ-, wobei A C2- bis C5-Alkylen bedeutet und m eine Zahl ist, die so gewählt ist, dass das Molekulargewicht von -(A-O)ₘ- mindestens 200 g/mol ist; Polycarbonatgruppen; Polyestergruppen; und Poly(meth)acrylatgruppen. Eine bevorzugte Spacergruppe ist alkoxyliertes Glycerin, z.B. ethoxyliertes Glycerin, propoxyliertes Glycerin und ethoxyliertes/propoxyliertes Glycerin.

Zu den aminischen Härtern, im Folgenden auch Aminhärter, zählen beispielsweise aliphatische und cycloaliphatische Monoamine und Polyamine, aromatische und araliphatische Monoamine und Polyamine sowie polymere Amine, z. B. Aminoplaste und Polyamidoamine. Aminhärter vernetzen Polymere mit Monothiocarbonatgruppen durch Reaktion der primären oder sekundären Aminofunktionen der Polyamine mit den Thiocarbonatgruppen unter Ausbildung von Urethanfunktionen. Bevorzugte Polyaminhärter weisen im Mittel wenigstens zwei primäre oder sekundäre Aminogruppen pro Molekül, z.B. zwei, drei oder vier primäre oder sekundäre Aminogruppen pro Molekül, auf. Sie können auch zusätzlich ein oder mehrere tertiäre Aminogruppen enthalten. Geeignete Polyamine sind beispielsweise
- aliphatische Polyamine wie Ethylendiamin, 1,2- und 1,3-Propandiamin, Neopentandiamin, Hexamethylendiamin, Octamethylendiamin, 1,10-Diaminodecan, 1,12-Diaminododecan, Diethylentriamin, Triethylentetramin, Tetraethylenpentamin, 2,2-Dimethylpropylendiamin, Trimethylhexamethylendiamin, 1-(3-Aminopropyl)-3-aminopropan, 1,3-Bis-(3-aminopropyl)propan, 4-Ethyl-4-methylamino-1-octylamin und dergleichen;
- cycloaliphatische Diamine wie 1,2-Diaminocyclohexan, 1,2-, 1,3-, 1,4-Bis(aminomethyl)cyclohexan, 1-Methyl-2,4-diaminocyclohexan, N-Cyclohexylpropylen-1,3-diamin, 4-(2-Aminopro-pan-2-yl)-1-methylcyclohexan-1-amin, Isophorondiamin, 4,4'-Diaminodicyclohexylmethan, 3,3'-Dimethyl-4,4'-diaminodicyclohexylmethan, 3,3',5,5'-Tetramethyl-4,4'-diaminodicyclohexylmethan, 4,8-Diamino-tricyclo[5.2.1.0]decan, Norbornandiamin, Menthandiamin, Menthendiamin und dergleichen;
- aromatische Diamine wie Toluylendiamin, Xylylendiamin, insbesondere meta-Xylylendiamin (MXDA), Bis(4-aminophenyl)methan (MDA oder Methylendianilin), Bis(4-aminophenyl)sulfon (auch als DADS, DDS oder Dapson bekannt) und dergleichen;
- cyclische Polyamine wie Piperazin, N-Aminoethylpiperazin und dergleichen;
- Polyetheramine, insbesondere difunktionelle und trifunktionelle primäre Polyetheramine auf der Basis von Polypropylenglykol, Polyethylenglykol, Polybutylenoxid, Poly-(1,4-butandiol), Poly-Tetrahydrofuran (Poly-THF) oder Polypentylenoxid, z. B. 4,7,10-Trioxatridecan-1,3-diamin, 4,7,10-Trioxatridecan-1,13-diamin, 1,8-Diamino-3,6-dioxaoctan (XTJ-504, Fa. Huntsman), 1,10-Diamino-4,7-dioxadecan (XTJ-590, Fa. Huntsman), 1,12-Diamino-4,9-dioxadode-can (Fa. BASF SE), 1,3-Diamino-4,7,10-trioxatridecan (Fa. BASF SE), primäre Polyetheramine auf der Basis von Polypropylenglykol mit einer mittleren Molmasse von 230 wie z.B. Polyetheramine D 230 (Fa. BASF SE) oder Jeffamine^{®} D 230 (Fa. Huntsman), difunktionelle, primäre Polyetheramine auf der Basis von Polypropylenglykol mit einer mittleren Molmasse von 400, z. B. Polyetheramine D 400 (Fa. BASF SE) oder Jeffamine^{®} XTJ 582 (Fa. Huntsman), difunktionelle, primäre Polyetheramine auf Basis von Polypropylenglykol mit einer mittleren Molmasse von 2000 wie z. B. Polyetheramine D 2000 (Fa. BASF SE), Jeffamine^{®} D2000 oder Jeffamine^{®} XTJ 578 (jeweils Fa. Huntsman), difunktionelle, primäre Polyetheramine auf der Basis von Propylenoxid mit einer mittleren Molmasse von 4000 wie z. B. Polyetheramin D 4000 (Fa. BASF SE), trifunktionelle, primäre Polyetheramine hergestellt durch Reaktion von Propylenoxid mit Trimethylolpropan, gefolgt durch eine Aminierung der endständigen OH-Gruppen mit einer mittleren Molmasse von 403 wie z.B. Polyetheramine T 403 (Fa. BASF SE) oder Jeffamine^{®} T 403 (Fa. Huntsman), trifunktionelle, primären Polyetheramin, hergestellt durch Reaktion von Propylenoxid mit Glycerin, gefolgt durch eine Aminierung der endständigen OH-Gruppen mit einer mittleren Molmasse von 5000 wie z. B. Polyetheramine T 5000 (Fa. BASF SE) oder Jeffamine^{®} T 5000 (Fa. Huntsman), aliphatische Polyetheramine, die aus einem mit Propylenoxid gepfropften Polyethylenglykol aufgebaut sind und eine mittlere Molmasse von 600 aufweisen, wie z. B. Jeffamine^{®} ED-600 bzw. Jeffamine^{®} XTJ-501 (Fa. Huntsman), aliphatische Polyetheramine, die aus einem mit Propylenoxid gepfropften Polyethylenglykol aufgebaut sind und eine mittlere Molmasse von 900 aufweisen, wie z. B. Jeffamine^{®} ED-900 (Fa. Huntsman), aliphatische Polyetheramine, die aus einem mit Propylenoxid gepfropften Polyethylenglykol aufgebaut sind und eine mittlere Molmasse von 2000 aufweisen, wie z.B. Jeffamine^{®} ED-2003 (Fa. Huntsman), difunktionelle, primäre Polyetheramin, hergestellt durch Aminierung eines mit Propylenoxid gepfropften Diethylenglykols mit einer mittleren Molmasse von 220 wie z. B. Jeffamine^{®} HK-511 (Fa. Huntsman), aliphatische Polyetheramine auf der Basis eines Copolymers aus Poly(tetramethylenether-glykol) und Polypropylenglykol mit einer mittleren Molmasse von 1000 wie z. B. Jeffamine^{®} XTJ-542 (Fa. Huntsman), aliphatische Polyetheramine auf der Basis eines Copolymers aus Poly(tetramethylenetherglykol) und Polypropylenglykol mit einer mittleren Molmasse von 1900 wie z. B. Jeffamine^{®} XTJ-548 (Fa. Huntsman), aliphatische Polyetheramine auf der Basis eines Copolymers aus Poly(tetramethylenetherglykol) und Polypropylenglykol mit einer mittleren Molmasse von 1400 wie z. B. Jeffamine^{®} XTJ-559 (Fa. Huntsman), Polyethertriamine auf der Basis eines mit Butylenoxid gepfropften mindestens dreiwertigen Alkohols mit einer mittleren Molmasse von 400 wie z. B. Jeffamine^{®} XTJ-566 (Fa. Huntsman), aliphatische Polyetheramine, hergestellt durch Aminierung von mit Butylenoxid aufgepfropften Alkoholen mit einer mittleren Molmasse von 219 wie z. B. Jeffamine^{®} XTJ-568 (Fa. Huntsman), Polyetheramine auf der Basis von Pentaerythrit und Propylenoxid mit einer mittleren Molmasse von 600 wie z. B. Jeffamine^{®} XTJ-616 (Fa. Huntsman), Polyetheramine auf der Basis von Triethylenglykol mit einer mittleren Molmasse von 148, z.B. Jeffamine^{®} EDR-148 (Fa. Huntsman), difunktionelle, primäre Polyetheramine, hergestellt durch Aminierung eines mit Propylenoxid gepfropften Ethylenglykols mit einer mittleren Molmasse von 176 wie z. B. Jeffamine^{®} EDR-176 (Fa. Huntsman) sowie Polyetheramine, hergestellt durch Aminierung von Poly-Tetrahydrofuran (Poly-THF) mit einer mittleren Molmasse von 250, z. B. PolyTHF-Amin 350 (Fa. BASF SE) und Mischungen dieser Amine;
- Polyamidoamine (Amidopolyamine), die durch die Reaktion von dimeren Fettsäuren (z. B. dimere Linolsäure) mit niedermolekularen Polyaminen wie Diethylentriamin, 1-(3-Aminopro-pyl)-3-aminopropan oder Triethylentetramin oder anderen Diaminen wie den zuvor genannten aliphatischen oder cycloaliphatischen Diaminen erhältlich sind;
- Addukte, die durch Umsetzung von Aminen, insbesondere Diaminen, mit einem Unterschuss an Epoxidharz bzw. Reaktivverdünner erhältlich sind, wobei man vorzugsweise solche Addukte einsetzt, worin etwa 5 bis 20 % der Epoxidgruppen mit Aminen, insbesondere Diaminen, umgesetzt worden sind;
- Phenalkamine, wie aus der Epoxidchemie bekannt;
- Mannichbasen, welche z. B. durch Kondensation von Polyaminen, vorzugsweise Diethylentriamin, Triethylentetramin, Isophorondiamin, 2,2,4- bzw. 2,4,4-Trimethylhexamethylendiamin, 1,3-und 1,4-Bis(aminomethyl)cyclohexan mit Aldehyden, vorzugsweise Formaldehyd und ein- oder mehrwertigen Phenolen mit mindestens einer aldehydreaktiven Kernstelle, z.B. die verschiedenen Kresole und Xylenole, p-tert.-Butylphenol, Resorcin, 4,4'-Dihydroxydiphenylmethan, 4,4'-Dihydroxydiphenyl-2,2-propan, vorzugsweise aber Phenol, hergestellt werden;
   sowie Mischungen der vorgenannten Aminhärter, insbesondere Mischungen von difunktionellen Aminen aus der Gruppe der aliphatischen, cycloaliphatischen und aromatischen Amine mit den vorgenannten Polyetheraminen.

Bevorzugte aminische Härter sind aliphatische Polyamine, insbesondere 2,2-Dimethylpropylendiamin, aromatische Diamine, insbesondere m-Xylylendiamin (MXDA) und cycloaliphatische Diamine, insbesondere Isophorondiamin, N-Cyclohexylpropylen-1,3-diamin und 4,4'-Diaminodicyclohexylmethan (Dicykan). Bevorzugt sind auch difunktionelle oder trifunktionelle primäre Polyetheramine auf der Basis von Polypropylenglykol, wie z. B. Jeffamine^{®} D 230 oder Jeffamine^{®} T 403. Besonders bevorzugt sind Polyamine, bei denen eine hohe Beweglichkeit und eine geringe sterische Hinderung rund um die Aminogruppe vorherrschen, z.B. 4,9-Dioxadode-can-1,12-diamin, 4,7,10-Trioxatridecan-1,13-diamin, PolyTHF Amin 350 (BASF SE).

Bevorzugt sind auch Mischungen der als bevorzugt genannten Amine, beispielsweise Mischungen, die 2,2-Dimethylpropylenamin und Isophoronamin enthalten.

Bevorzugt sind auch Härterverbindungen B mit geringem sterischen Anspruch, z.B. ohne quaternäre C-Atome. Beispiele dafür sind alpha,omega-Diamino-n-alkane, wobei die Alkylkette mit Sauerstoffatomen unterbrochen sein kann (z.B. 4,9-Dioxa-dodecan-1,12-diamin) oder Polyethylenimin (z.B. Luprasol^{®} FG).

Bevorzugt sind auch Klebstoffmischungen mit möglichst langer offener Zeit, die man dadurch erhält, dass man, Härterverbindungen B verwendet, bei denen die funktionellen Gruppen in maskierter, latent reaktiver Form vorliegen, z.B. als Oxazolidine, Aldimine, Ketimine oder Enamine.

Geeignete Härter sind auch Aminosäuren, z. B. Lysin, Arginin, Glutamin und Asparagin und deren Stereoisomere und deren Mischungen.

Es können natürlich auch Mischungen unterschiedlicher Härter eingesetzt werden, z.B. Mischungen eines oder mehrerer aminischer Härter oder Mischungen eines oder mehrerer aminischer Härter mit einer oder mehreren Aminosäuren.

In den erfindungsgemäßen Klebstoffzusammensetzungen beträgt die Gesamtmenge an Härtern vorzugsweise 0,1 Gew.-% bis 50 Gew.-%, häufig 0,5 bis 40 Gew.-% und insbesondere 1 bis 30 Gew.-%, bezogen auf die Gesamtmenge an Monothiocyclocarbonatverbindungen plus eingesetzten Härtern.

Die Härtung der Klebstoffzusammensetzung kann thermisch durch Erwärmen des Gemischs aus Verbindung A und Härterverbindung B auf eine Temperatur oberhalb der Mischtemperatur erfolgen. Die Härtung kann auch bei niedrigeren Temperaturen erfolgen. Typischerweise erfolgt die Härtung der erfindungsgemäßen Klebstoffzusammensetzungen bei Temperaturen im Bereich von -10°C bis 150 °C, vorzugsweise im Bereich von 0 bis 100 °C und insbesondere im Bereich von 10 bis 70 °C. Besonders vorteilhaft ist die Härtung bei Temperaturen von 20-30°C. Welche Temperatur geeignet ist, hängt von den jeweiligen Härtern und der gewünschten Härtungsgeschwindigkeit ab und kann im Einzelfall vom Fachmann beispielsweise anhand einfacher Vorversuche ermittelt werden. Im unteren Temperaturbereich (5 bis ca. 35 °C), der ja der meist vorherrschenden Umgebungstemperatur entspricht, reicht es selbstverständlich aus, Verbindung A und Härterverbindung B zu mischen. Alternativ erfolgt die Härtung vorzugsweise mikrowelleninduziert.

Die Zweikomponentenklebstoffzusammensetzungen können auch einen oder mehrere geeignete Katalysatoren für die Aushärtung enthalten, die sich in bekannter Weise nach der Art der reaktiven funktionellen Gruppen F richten. Die Katalysatoren werden, sofern erwünscht, in Anteilen von 0,01 Gew.-% bis etwa 10 Gew.-%, bezogen auf die Summe der Verbindungen A und B eingesetzt. In einer Ausgestaltung werden keine Katalysatoren benötigt, insbesondere bei Härtern, die als funktionelle Gruppen Aminogruppen aufweisen, d.h., der Gehalt an Katalysatoren in der Zusammensetzung beträgt dann weniger als 0,01 Gew.-%. Katalysatoren werden bevorzugt dann eingesetzt, wenn der Härter zusätzliche reaktive Gruppen F aufweist, die von Aminogruppen verschieden sind.

Bevorzugt eingesetzte Katalysatoren sind basische Katalysatoren, besonders bevorzugt organische Amine und organische Phosphine. Unter den organischen Aminen sind Amidinbasen, wie beispielsweise 1,8-Diazabicyclo[5.4.0]undec-7-en (DBU) und 1,5-Diazabicyclo[4.3.0]non-5-en (DBN), Mono-C₁-C₆-alkyl-, Di-C₁-C₆-alkyl- und Tri-C₁-C₆-alkylamine, insbesondere Triethylamin und tert.-Butylamin, bevorzugt. Unter den organischen Phosphinen sind Trialkylphosphine und Triarylphosphine bevorzugt, beispielsweise Tri-n-butylphosphin und Triphenylphosphin. Die Katalysatoren können selbstverständlich auch als Mischungen eingesetzt werden, gegebenenfalls in Kombination mit Tri-C₁-C₆-alkylammoniumhalogeniden und Kupfersalzen, zum Beispiel Triphenylphosphin in Kombination mit einem Tri-C₁-C₆-alkylammoniumhalogenid und einem Kupfersalz, z. B. Kupfer(I)-chlorid, Kupfer(I)-bromid, Kupfer(II)-chlorid oder Kupfer(II)-sulfat.

Die Härterverbindung B des Zweikomponentenklebstoffs kann eine Verbindung B1 sein, die zusätzlich zu der mindestens einen funktionellen Gruppe, ausgewählt aus primären Amingruppen und sekundären Amingruppen, noch mindestens eine mit SH-Gruppen reaktive ethylenisch ungesättigte Bindung aufweist. Molekulargewichte und Anzahl an funktionellen Gruppen einschließlich der mit SH-Gruppen reaktiven Gruppen, sind vorzugsweise wie für Verbindung B beschrieben. Vorzugsweise hat Verbindung B1 ein, zwei oder drei primäre oder sekundäre Amingruppen, besonders bevorzugt mindestens eine oder genau eine primäre Amingruppe. Vorzugsweise hat Verbindung B1 ein, zwei oder drei mit SH-Gruppen reaktive Gruppen. Die mit SH-Gruppen reaktive Gruppe von B1 ist vorzugsweise eine nicht-aromatische C-C-Doppelbindung oder eine C-C-Dreifachbindung. Vorzugsweise enthält Verbindung B1 keine andere funktionelle Gruppen ausser mit SH-Gruppen reaktive Bindungen, primäre oder sekundäre Amingruppen, Hydroxygruppen, Carbonsäureestergruppen und Ethergruppen. Besonders bevorzugt ist Verbindung B1 eine Verbindung mit einer einzigen primären Aminogruppe und einer einzigen nicht-aromatischen C-C-Doppelbindung.

Geeignete Verbindungen B1 sind z.B. Aminoalkylvinylether mit 1 bis 10 C-Atomen in der Alkylgruppe (z.B. Aminopropylvinylether) oder Allylamin:

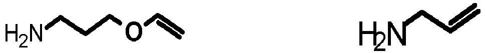

Der Zweikomponentenklebstoff kann optional in der ersten Komponente, in der zweiten Komponente und/oder in einer weiteren Komponente mindestens eine Verbindung C enthalten, welche eine Anzahl n_{C3} an mit SH-Gruppen reaktiven funktionellen Gruppen, z.B. Gruppen mit ethylenisch ungesättigten Mehrfachbindungen, vorzugsweise ethylenisch ungesättigten Doppelbindungen, oder Epoxygruppen enthält und n_{C3} eine ganze Zahl größer oder gleich 1 ist. Besonders bevorzugt sind (meth)acrylische, allylische und vinylische C-C-Doppelbindungen. Bevorzugt ist auch, dass eine Mischung eingesetzt wird von mindestens einer Verbindung mit mindestens einer Gruppe mit ethylenisch ungesättigten Mehrfachbindungen und mindestens einer Verbindung mit mindestens einer Epoxygruppe.

Die Verbindungen C können ein Molekulargewicht von bis zu 500.000 g/mol haben. Im Fall von Polymeren handelt es sich dabei um das gewichtsmittlere Molekulargewicht, gemessen durch Gelpermeationschromtatographie in THF mit Polystyrolstandard. Vorzugsweise haben die Verbindungen C ein Molekulargewicht von bis zu 1000 g/mol, besonders bevorzugt von 60 g/mol bis 500 g/mol.

Die Verbindungen C können beispielsweise bis zu 1000, insbesondere bis zu 500, vorzugsweise bis zu 100 mit SH-Gruppen reaktive Gruppen aufweisen. Besonders bevorzugt weisen die Verbindungen C zwei oder drei mit SH-Gruppen reaktive Gruppen auf. Die mit SH-Gruppen reaktiven ethylenisch ungesättigten Mehrfachbindungen können nicht-aromatische C-C-Doppelbindungen oder C-C Dreifachbindungen sein, besonders bevorzugt sind nicht-aromatische C-C-Doppelbindungen. Eine Dreifachbindung kann zweimal mit SH-Gruppen reagieren. Zuerst kann sich eine SH-Gruppe an eine Dreifachbindung addieren, wodurch die Dreifachbindung zu einer Doppelbindung wird. Die gebildete Doppelbindung kann mit einer weiteren SH-Gruppe reagieren. Deshalb entspricht eine Dreifachbindung zwei mit SH-Gruppen reaktiven ethylenisch ungesättigten Bindungen. Verbindungen C mit nur einer ungesättigten Gruppe werden auch als Monomere bezeichnet, Verbindungen C mit mindestens zwei ungesättigten Gruppen werden auch als Oligomere bezeichnet. Solche Oligomere haben vorzugsweise 2 bis 10, insbesondere 2 oder 3 ungesättigte Gruppen.

Bevorzugte Verbindungen C sind Verbindungen, welche mindestens eine der folgenden Gruppen aufweisen:
Vinylgruppe CH₂=CH- ; Vinylengruppe -CH=CH-, ungesättigte Carbonylgruppe CH₂=CR-C(=O)-mit R= H oder Alkyl; Acrylgruppe CH₂=CH-C(=O)-O-; Methacrylgruppe CH₂=C(CH₃)-C(=O)-O, Acrylamidgruppe CH₂=CH-C(=O)-N, Cyanacrylgruppe CH₂=C(CN)-C(=O)-O, Methylenmalonatgruppe CH₂=C[C(=O)-O]₂ Vinylen-1,3-dicarbonylgruppe CH₂=C[C(=O)-]₂ 1,4-Dicarboxyalkylen-Gruppe
-OC(=O)-CH =CH-C(=O)O-], Allylgruppe CH₂=CH-CH₂-, insbesondere Allylether CH₂=CH-CH₂-O-, Maleimidgruppe oder Crotonylgruppe. Bevorzugte monofunktionelle Verbindungen C sind Acryl- und Methacrylverbindungen, Vinylesters, z.B. Vinylacetat, Vinylether, Vinyllactame, z.B. N-Vinylpyrrolidon, Vinylaromaten, z.B. Styrol, Vinylhalogenide z.B.Vinylchlorid, Vinylfluorid, Olefine mit einer einzigen C-C-Doppelbindung, z.B. Ethylen, Propylen.

Im Folgenden werden gelegentlich die Bezeichnung "(Meth)acryl..." und ähnliche Bezeichnungen als abkürzende Schreibweise verwendet für "Acryl... oder Methacryl...". In der Bezeichnung Cx-Alkyl(meth)acrylat und analogen Bezeichnungen bedeutet x die Anzahl der C-Atome der Alkylgruppe.

Besonders bevorzugte Verbindungen C sind (Meth)acrylverbindungen und Vinylether. (Meth)acrylverbindungen sind z.B. (Meth)acrylester, insbesondere Alkyl- oder Hydroxyalkyl(meth)acrylate, (Meth)acrylnitril, oder (Meth)acrylsäue. Vinylether sind z.B. Vinylalkylether. Alkylgruppen haben vorzugsweise 1 bis 20, besonders bevorzugt 1 bis 8 C-Atome.

Verbindungen C mit Dreifachbindung sind z.B. Alkine wie z.B. Acetylen oder Propin; Propargylalkohol, Ether von Propargylalkohol, Ester von Popargylalkohol, Propargylamin oder Amide von Propargylamin.

Bevorzugte Verbindungen C sind insbesondere Verbindungen mit mindestens zwei (Meth)acrylatgruppen, Verbindungen mit mindestens zwei Vinylgruppen, Olefine mit mindestens zwei C-C Doppelbindungen, mit zwei oder mehr ethylenisch ungesättigten Gruppen substituierte Polyester, mit zwei oder mehr ethylenisch ungesättigten Gruppen substituierte Cyanurate oder mit zwei oder mehr ethylenisch ungesättigten Gruppen substituierte Isocyanurate. Olefine mit zwei oder mehr C-C Doppelbindungen sind z.B. Butadien, Cyclooctadien, Cyclododecatrien, Isopren, Limonen, Divinylcyclohexan, Polybutadien oder Polyisopren. Verbindungen C mit mindestens zwei (Meth)acrylgruppen sind z.B. (Meth)acrylsäureestern von polyfunktionellen Alkoholen oder von alkoxylierten polyfunktionellen Alkoholen. Beispiele für polyfunktionelle Alkohole sind bifunktionelle Alkohole, wie Ethylenglykol, Diethylenglykol, Triethylenglykol, Propylenglykol, Dipropylenglykol, Tripropylenglykol, Butandiol, Pentandiol, Hexandiol, Neopentylglkcol, alkoxylierte Phenole, wie ethoxylierte oder propoxyliertes Bisphenole, Cyclohexandimethanol. Trifunktionelle und höher funktionelle Alkohole sind z.B. Glycerin, Trimethylolpropan, Butantriol, Trimethylolethan, Pentaerythritol, Ditrimethylolpropan, Dipentaerythritol, Sorbitol, Mannitol und die entsprechenden alkoxylierten, insbesondere ethoxylierten und/oder propoxylierten Alkohole.

Geeignete Verbindungen C mit mehr als zwei ungesättigten Gruppen sind auch (Meth)acrylsäureester von Polyesterolen. Geeignete Polyesterole sind z.B. solche, die herstellbar sind durch Verestern von Polycarbonsäuren, vorzugsweise von Dicarbonsäuren mit Polyolen, vorzugsweis mit Diolen. Bevorzugte Dicarbonsäuren sind Bernsteinsäure, Glutarsäure, Adipinsäure, Sebacinsäure, ortho-Phthalsäure und deren Isomere und hydrierten Produkte sowie die veresterbaren oder umesterbaren Derivate der genannten Säuren, z.B. deren Anhydride und Alkylester. Geeignete Dicarbonsäuren sind auch Maleinsäure, Fumarsäure und Tetrahydrophthalsäure oder deren Anhydride. Bevorzugte Polyole sind Ethylenglykol, 1,2- und 1,3-Propylenglykol, Butan-1,4-diol, Hexan-1,6-diol, Neopentylglykol, Cyclohexandimethanol und Polyglykols von Ethylenglykol und/oder Propylenglykol.

Geeignete Verbindungen C mit zwei oder mehr ethylenisch ungesättigten Gruppen sind auch Epoxid(meth)acrylate und Urethan(meth)acrylate. Epoxid(meth)acrylate sind z.B. diejenigen herstellbar durch Reaktion von epoxidierten Olefinen oder von Poly-, Mono- oder Diglycidylethern, wie Bisphenol A diglycidylether, mit (Meth)acrylsäure. Urethan(meth)acrylate sind z.B. die Reaktionsprodukte von Hydroxyalkyl(meth)acrylaten mit Poly- oder Diisocyanaten.

Verbindungen C mit zwei oder mehr ethylenisch ungesättigten Gruppen sind auch ungesättigte Polyester, insbesondere solche mit C-C-Doppelbindungen aus Maleinsäure-, Itaconsäure- oder Fumarsäureinheiten. Verbindungen C mit zwei oder mehr ethylenisch ungesättigten Gruppen sind auch solche mit mindestens zwei Vinylgruppen, z.B. Divinylether wie Diethylenglycoldivinylether oder Triethylenglycoldivinylether oder Divinylsulfon. Geeignete Verbindungen C mit zwei oder mehr ethylenisch ungesättigten Gruppen sind auch:

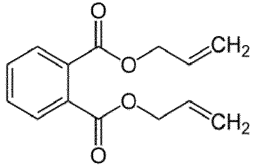

Diallyl-ortho-phthalat

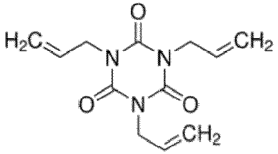

Triallylisocyanurate

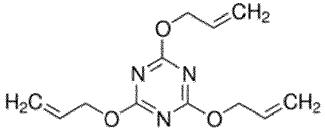

### Triallylcyanurat

Besonders bevorzugte Verbindungen C sind (Meth)acrylsäureester von polyfunktionellen Alkoholen, oder Verbindungen mit Vinylethergruppen oder ungesättigte Polyester, insbesondere Tri-methylolpropantri(meth)acrylat und Alkandioldi(meth)acrylate von C2- bis C8-Alkandiolen.

Bevorzugte Verbindungen C sind insbesondere polyfunktionellen (Meth)acrylester die erhältlich sind unter den Bezeichnungen Laromer^{®} (BASF), Sartomer^{®} (Arkema) oder Miramer^{®} (Miwon); oder Methacrylamide.

Geeignete Verbindungen C sind auch solche mit mindestens einer Epoxygruppe, z.B. Verbindungen erhältlich durch Reaktion von Verbindungen mit mindestens einer Alkoholgruppe mit Epichlorohydrin.

Verbindungen C mit einer Epoxygruppe sind beispielsweise Epichlorohydrin oder Derivate davon, wobei das Chlorid des Epichlorohydrins ersetzt ist durch eine Hydroxygruppe (Glycidol), durch eine Ethergruppe (Glycidylether), durch eine Estergruppe (Glycidylester) oder durch eine Aminogruppe (Glycidylamin).

Beispiele von Verbindung C mit mindestens zwei Epoxygruppen sind die Diglycidylether von Bisphenol A oder von Bisphenol F oder von Bisphenol S, die Diglycidylether von hydriertem Bisphenol A oder von hydriertem Bisphenol F und Diglycidylether von aliphatischen Diolen, z.B. Diglycidylether von Polyalkoxylendiolen. Geeignet sind auch Oligoglycidylether von Aligoalkoholen. Weitere Beispiele sind auch Epoxyharze, erhältlich indem die Verbindungen mit mindestens zwei Alkoholgruppen im Überschuss verwendet werden in Bezug auf Epichlorhydrin. In derartigen Epoxyharzen ist der Polymerisationsgrad der Verbindung mit mindestens zwei Alkoholgruppen vorzugsweise 2 bis 25, insbesondere 2 bis 10.

Weitere Beispiele für Verbindung C sind epoxidierte Fettsäure, epoxidierte Fettsäureester und epoxidierte Fettalkohole, welche jeweils mindestesn zwei Epoxygruppen aufweisen. Weitere Beispiele für Verbindung C sind Tetraglycidylmethylenedianiline (TGMDA), Triglycidylaminophenol und Triglycidylisocyanurate (siehe unten)

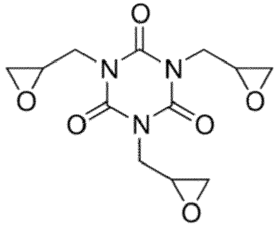

Weitere Verbindungen C mit mehr als seiner Epoxygruppe sind erhältlich durch Polymerisation oder Copolymerisation von Glycidyl(meth)acrylate oder von Glycidylvinylether.

Bei der Reaktion der Monothiocarbonatgruppen der Verbindung A mit den funktionellen Gruppen der Verbindung B des Zweikomponentenklebstoffs entstehen zunächst Verbindungen mit SH-Gruppen. Diese SH-Gruppen sind sehr reaktiv und können weiterreagieren, z.B. mit den SH-reaktiven funktionellen Gruppen der Verbindungen B oder C. Je nach Anzahl der Funktionalitäten in den Verbindungen A, B, und C können dabei vernetzte Polymere entstehen oder Polymere mit über ein S-Atom verknüpften Seitengruppen.

Wenn der Zweikomponentenklebstoff keine Verbindungen mit SH-reaktiven Gruppen enthält, können die SH-Gruppen mit Luftsauerstoff oxidieren und z.B. Disulfidbrücken bilden.

Vorzugsweise ist die Anzahl an funktionellen Gruppen der Verbindungen A, B und C derart, dass sich Polymere, besonders bevorzugt vernetzte Polymere bilden. Besonders bevorzugt ist die Summe der Anzahl an Monothiocarbonatgruppen der Verbindung A und der damit reaktiven funktionellen Gruppen der Verbindung B größer oder gleich 3, insbesondere größer oder gleich 4 und es ist eine Verbindung C vorhanden mit größer oder gleich 2 mit SH-Gruppen reaktiven funktionellen Gruppen.

Der Zweikomponentenklebstoff enthält Härterverbindung B vorzugsweise in einer Menge, sodass die Menge n_{B} an funktionellen Gruppen der Härterverbindung B von 50 mol% bis 150 mol%, vorzugsweise von 80 mol% bis 120 mol% beträgt, bezogen auf die Menge n_{A} an Cyclo-thiocarbonatgruppen der Verbindung A. Der Zweikomponentenklebstoff enthält Verbindung C vorzugsweise in einer Menge, sodass die Menge der reaktiven Doppelbindungen n_{C3} von 0 bis 120 mol%, vorzugsweise von 90 bis 110 mol% beträgt, bezogen auf die Menge n_{A} an Cyclothiocarbonatgruppen.

Zur Lagerung bis zu Anwendung können die Verbindungen A und B in separaten Komponenten gehalten werden, die erst kurz vor der Anwendung vermischt werden. Verbindung C kann entweder ebenfalls in einer separaten Komponente oder bereits vermischt mit Verbindung A und/oder Verbindung B vorliegen. Bei einer Mischung von Verbindungen B und C in einer Komponente liegt Verbindung B vorzugsweise in maskierter, latent reaktiver Form vor.

Die Anwendung und Härtung des Zweikomponentenklebstoffs erfolgt vorzugsweise bei Temperaturen von -20 bis 250°C, besonders bevorzugt bei 20 bis 100°C. Der erfindungsgemäße Zweikomponenten-Klebstoff ist in der Lage, in kurzer Zeit und insbesondere mit Aminhärtern bereits bei Raumtemperatur hohe Bindekräfte aufzubauen. Bevorzugt ist deshalb ein Klebeverfahren, wobei die Verklebung bei Temperaturen kleiner oder gleich 30°C, vorzugsweise ohne Erwärmung erfolgt.

Neben den vorgenannten Bestandteilen kann die Klebstoffzusammensetzung die hierfür üblichen Additive enthalten. Die Wahl geeigneter herkömmlicher Additive für die erfindungsgemäße Zusammensetzung hängt vom jeweiligen Verwendungszweck ab und kann im Einzelfall vom Fachmann bestimmt werden.

Geeignete Additive umfassen beispielsweise Katalysatoren, Initiatoren, Inhibitoren, Antioxidantien, UV-Absorber/Lichtstabilisatoren, Metalldeaktivatoren, Antistatika, Verstärkungsstoffe, Füllstoffe, Antifoggingmittel, Biozide, Weichmacher, Gleitmittel, Emulgatoren, Farbmittel, Pigmente, Rheologiemittel, Adhäsionsregulatoren, optische Aufheller, Flammschutzmittel, Antitropfmittel, Nukleierungsmittel, Netzmittel, Verdicker, Schutzkolloide, Entschäumer, Tackifier, Lösungsmittel und Reaktivverdünner sowie Gemische davon.

Vorzugsweise enthält der Klebstoff mindestens einen Katalysator für die Katalysierung der Reaktion der Cyclocarbonatgruppen der Verbindung A mit den funktionellen Gruppen der Härterverbindung B und/oder mindestens einen Katalysator oder Initiator für die Katalysierung oder Initiierung der Reaktion von SH-Gruppen miteinander oder der Reaktion von SH-Gruppen mit den hiermit reaktiven funktionellen Gruppen, z.B. mit den ethylenisch ungesättigten Doppelbindungen.

Geeignete Katalysatoren für die Katalysierung der Reaktion der Cyclocarbonatgruppen der Verbindung A mit den funktionellen Gruppen der Härterverbindung B sind z.B. basische Katalysatoren wie tertiäre Amine, Pyridine, Guanidine oder organische Phosphine, z.B. 1,8-Diazabicyclo-[5.4.0]undec-7-en (DBU), 1,5-Diazabicyclo[4.3.0]non-5-en (DBN); Tri-C1-C6-alkylamine, besonders bevorzugt Triethylamin. Organische Phosphine sind beispielsweise Trialkylphosphine, wie z.B. Tri-n-butylphosphin oder Triarylphosphine wie z.B. Triphenylphosphin.

Geeignete Katalysatoren oder Initiatoren für die Katalysierung oder Initiierung der Reaktion von SH-Gruppen miteinander oder der Reaktion von SH-Gruppen mit ethylenisch ungesättigten Doppelbindungen sind beispielsweise radikalbildende Verbindungen, z.B. Azoinitiatoren wie AIBN, organische Peroxidverbindungen, Redoxpaare (H2O2, tert.-Butylperoxid, Asccorbinsäure) sowie Photoinitatoren und Sauerstoff selbst. Bei aktivierten Doppelbindungen (sogenannten Michael-Systemen) kann die Reaktion der Doppelbindung nucleophil erfolgen und daher katalysiert werden z.B. durch tertiäre Amine, Guanidine, Pyridine, Phosphine etc.. Bei nicht aktivierten Doppelbindungen kann die Reaktion der Doppelbindung radikalisch erfolgen und kann thermisch mit einem Radikalstarter oder photochemisch mit einem Photoinitiator initiiert werden.

Die gegebenenfalls verwendeten Lichtstabilisatoren/UV-Absorber, Antioxidantien und Metalldeaktivatoren weisen vorzugsweise eine hohe Migrationsstabilität und Temperaturbeständigkeit auf. Sie sind beispielsweise aus den Gruppen a) bis t) ausgewählt. Die Verbindungen der Gruppen a) bis g) und i) stellen Lichtstabilisatoren/ UV-Absorber dar, während Verbindungen j) bis t) als Stabilisatoren wirken.
a) 4,4-Diarylbutadiene,
b) Zimtsäureester,
c) Benzotriazole,
d) Hydroxybenzophenone,
e) Diphenylcyanacrylate,
f) Oxamide,
g) 2-Phenyl-1,3,5-triazine,
h) Antioxidantien,
i) Nickelverbindungen,
j) sterisch gehinderte Amine,
k) Metalldesaktivatoren,
l) Phosphite und Phosphonite,
m) Hydroxylamine,
n) Nitrone,
o) Aminoxide,
p) Benzofuranone und Indolinone,
q) Thiosynergisten,
r) Peroxid-zerstörende Verbindungen,
s) Polyamidstabilisatoren und
t) basische Costabilisatoren.

Der Zweikomponenten-Klebstoff ist vorzugsweise frei von Isocyanaten, d.h. er enthält vorzugsweise keine Isocyanatverbindungen als Härter.

Der Zweikomponenten-Klebstoff liegt vorzugsweise entweder in Form einer Lösung in einem organischen Lösungsmittel (z.B. THF, Aceton, Ethylacetat, Acetonitril, Toluol) vor, bevorzugt ist er aber lösemittelfrei. Lösemittelfrei bedeutet, dass weniger als 5 Gew.%, besonders bevorzugt weniger als 2 Gew.% oder kein organisches Lösungsmittel oder Wasser enthalten ist.

Die Komponenten des Klebstoffs sind zum Zeitpunkt der Vermischung bei 23°C vorzugsweise flüssig und haben vorzugsweise eine Nullviskosität kleiner 1000 Pa s, bevorzugt kleiner 500 Pa s, besonders bevorzugt kleiner 100 Pa s. Der Zweikomponenten-Klebstoff hat nach Vermischen der Komponenten im noch nicht ausgehärteten Zustand eine Nullviskosität bei 70°C, vorzugsweise auch bei 40 °C, besonders bevorzugt auch bei 23°C von vorzugsweise kleiner 3000 Pa s, oder kleiner 1500 Pa s, besonders bevorzugt kleiner 300 Pa s.

Der Zweikomponenten-Klebstoff hat eine Klebkraft von vorzugsweise grösser als 1,5 N/15 mm gemessen als Schälfestigkeit von zwei miteinander mit einer Klebstoffschicht von 3 µm und mit einem Anpressdruck von 3 bar verklebten Polyethylenterephthalatfolien nach 24 Stunden.

Gegenstand der Erfindung ist auch ein Klebeverfahren, wobei zwei Substrate miteinander verklebt werden und auf die Oberfläche mindestens eines der Substrate ein 2-Komponentenklebstoff aufgetragen wird, welcher mindestens eine der oben näher beschriebenen Verbindungen A mit cyclischen Monothiocarbonat-Einheiten als reaktive Komponente und Härterverbindung B enthält.

Die zu verklebenden Substrate können gleich oder voneinander verschieden sein und sind vorzugsweise ausgewählt aus Metall, Holz, Glas Kunststoffformkörpern, Kunststofffolien, Papier und Pappe.

Bevorzugte Anwendungen und bevorzugte Klebeverfahren sind die Verbundfolienkaschierung, die Glanzfolienkaschierung und die Kaschierung von Formkörpern, wie sie insbesondere bei der Möbelkaschierung oder bei der Kaschierung von Automobilinnenteilen eingesetzt wird, sowie die Anwendung als Strukturkleber.

Gegenstand der Erfindung ist auch ein Kaschierverfahren zur Herstellung von kaschierten Gegenständen, ausgewählt aus Glanzfolien, Verbundfolien und kaschierten Formkörpern, wobei man
a) ein erstes Substrat in Form einer ersten Folie zur Verfügung stellt,
b) ein zweites Substrat zur Verfügung stellt, ausgewählt aus Papier, einer zweiten Folie die zur ersten Folie gleich oder verschieden sein kann, und Formkörpern,
c) einen oben näher beschriebenen erfindungsgemäßen Zweikomponentenklebstoff zur Verfügung stellt und
d) den Zweikomponentenklebstoff (nach Vermischen der Komponenten) auf das erste Substrat und/oder auf das zweite Substrat aufträgt, optional trocknen lässt und das erste Substrat auf den das zweite Substrat aufkaschiert, wobei die Aufkaschierung unter Thermoaktivierung erfolgen kann.

Die erste Folie ist vorzugsweise ausgewählt aus Kunststofffolien und Aluminiumfolien, wobei die Kunststofffolien auch metallisiert sein können. Das Aufkaschieren erfolgt vorzugsweise unter Druck und/oder Temperaturerhöhung, insbesondere durch Thermoaktivierung. Wenigstens eines der Substrate kann auf der mit dem Klebstoff beschichteten Seite bedruckt oder metallisiert sein.

Gegenstand der Erfindung sind auch folienkaschierte Gegenstände, hergestellt nach dem erfindungsgemäßen Kaschierverfahren, wobei das Folienmaterial vorzugsweise ausgewählt ist aus der Gruppe bestehend aus Polyvinylchlorid, das auch Weichmacher enthalten kann, und thermoplastischem Polyolefin (TPO) und Kombinationen daraus.

Die benutzten Folien sind vielfach Kunststoffdekorfolien und können eine Oberflächenstruktur aufweisen. Diese Oberflächenstruktur auf der Kunststofffolie kann beispielsweise vor, während oder nach dem Verkleben eingeprägt werden.

Eine Oberflächenbehandlung der Foliensubstrate ist vor der Beschichtung mit dem Zweikomponentenklebstoff nicht unbedingt erforderlich. Bessere Ergebnisse können aber erhalten werden, wenn die Oberfläche der Foliensubstrate vor der Beschichtung modifiziert werden. Hierbei können übliche Oberflächenbehandlungen angewendet werden, z.B. Coronabehandlung zur Verstärkung der Haftwirkung. Vorzugsweise weist die Polymerfolie an der mit dem Klebstoff in Kontakt kommenden Oberfläche hydrophile Gruppen auf. Hydrophile Gruppen sind z.B. Sauerstoff enthaltende Gruppen beispielsweise OH-Gruppen oder Säuregruppen. Die hydrophilen Gruppen werden vorzugsweise erzeugt durch Coronabehandlung zur Verstärkung der Haftwirkung. Die Coronabehandlung oder andere Oberflächenbehandlungen werden in dem Maße durchgeführt, wie für eine ausreichende Benetzbarkeit mit der Beschichtungszusammensetzung erforderlich ist. Üblicherweise ist eine Coronabehandlung von ungefähr 10 Watt pro Quadratmeter und Minute für diesen Zweck ausreichend. Alternativ oder zusätzlich können optional auch noch Primer oder Zwischenschichten zwischen Foliensubstrat und Klebstoffbeschichtung und/oder Formkörpersubstrat verwendet werden.

Außerdem können die Folien, weitere, zusätzliche funktionale Schichten aufweisen, z. B. Barriereschichten, Druckschichten, Farb- oder Lackschichten oder Schutzschichten. Die funktionalen Schichten können sich dabei außen, d.h. auf der mit Klebstoff beschichteten Seite abgewandten Seite des Foliensubstrats oder innen, zwischen Foliensubstrat und Klebstoffschicht befinden.

Die Kaschierung von Formkörpern betrifft die Herstellung von Verbundkörpern durch dauerhafte Verklebung von großflächigen, biegsamen Folien auf festen (dreidimensional geformten, formstabilen, nicht flexiblen) Formkörpern als Substrat. Die biegsamen Folien sind insbesondere ausgewählt aus Polymerfolien und Metallfolien. Sie werden mit den festen Formkörpern, z.B. Formteilen aus Metall, lackiertem Metall, Holz, Holzwerkstoffen, Fasermaterialien oder Kunststoff verklebt. Bei den Formteilen kann es sich um Möbel oder um Möbelteile, d.h. um Bestandteile von Möbeln oder um Automobilinnenteile handeln.

In einer Ausführungsform handelt es sich bei den kaschierten Formkörpern um folienbeschichtete Möbel. Bei den erfindungsgemäß hergestellten folienbeschichteten Möbeln handelt es sich um Verbundkörper. Die Verbundkörper können zwischen Folie und Klebstoffschicht und/oder zwischen Substrat und Klebstoffschicht zusätzlich Primerschichten zur Haftverbesserung aufweisen. Die zu verklebenden Folien und Substrate können mit Haftvermittlern vorbehandelt sein. Aufgrund der bereits guten Hafteigenschaften erfindungsgemäßer Klebstoffe ist die Anwendung von Primern aber nicht unbedingt erforderlich. Bei den Möbelteilen kann es sich auch um Formteile handeln, welche aus synthetischen oder natürlichen Fasern oder Spänen aufgebaut sind, die durch ein Bindemittel zu einem Formteil gebunden sind. Die Formteile können eine beliebige Form haben. Besonders bevorzugt sind MDF-Platten (mitteldichte Holzfaserplatten).

Bei der Herstellung von folienkaschierten Formteilen für den Automobilbau erfolgt die Kaschierung auf ein für den Einbau in ein Automobil vorgesehenes Formteil. Bei den Formteilen kann es sich auch um Formteile handeln, welche aus synthetischen oder natürlichen Fasern oder Spänen aufgebaut sind, die durch ein Bindemittel zu einem Formteil gebunden sind; insbesondere sind auch Formteile aus Kunststoff, z. B. ABS, geeignet. Die Formteile können eine beliebige Form haben.

Als erstes Substrat besonders bevorzugte Folien sind Polymerfolien. Als Polymerfolie werden insbesondere biegsame flächige Kunststoffe in einer Dicke von 0.05 Millimeter bis 5 Millimeter, vorzugsweise von 0,25 bis 1 mm verstanden, die sich aufrollen lassen. Somit werden neben "Folien" im strengen Sinn von Dicken unter 1 mm, auch Abdichtungsbahnen, wie sie typischerweise zum Abdichten von Tunnels, Dächern oder Schwimmbädern in einer Dicke von typischerweise 1 bis 3 mm, in Spezialfällen sogar in einer Dicke bis maximal 5 mm, verwendet werden, verstanden. Derartige Kunststofffolien werden üblicherweise durch Streichen, Gießen, Extrusion oder besonders bevorzugt durch Kalandrieren hergestellt und sind typischerweise in Rollen kommerziell erhältlich oder werden vor Ort hergestellt. Sie können einschichtig oder mehrschichtig aufgebaut sein. Der Kunststoff der Polymerfolien ist vorzugsweise ein thermoplastischer Kunststoff, z.B. Polyester, wie Polyethylenterephthalat (PET), thermoplastische Polyolefine (TPO) wie Polyethylen, orientiertem Polypropylen (OPP), ungerecktes Polypropylen (CPP), Polyvinylchlorid, insbesondere Weich-PVC, Polyacetate, Ethylen/ Vinylacetat Copolymere (EVA), ASA (Acrylnitril/Styrol/Acrylsäureester Copolymere), PUR (Polyurethan), PA (Polyamid), Poly(meth)acrylate, Polycarbonate, oder deren Kunststofflegierungen, Zellophan, mit Metall, z. B. Aluminium, beschichtete (bedampfte) Polymerfolien (kurz : metallisierte Folien) oder Metallfolien, z. B. aus Aluminium. Die genannten Folien können beispielsweise auch mit Druckfarben bedruckt sein. Besonders bevorzugt sind Hart-PVC und thermoplastisches Polyethylenterephthalat (PET).

Die Beschichtung der Folien und Substrate mit dem Klebstoff kann nach üblichen Auftragsverfahren erfolgen, beispielsweise durch ein Sprüh-, Streich-, Rakel-, Stempel-, Walz- oder Gießauftragsverfahren. Bevorzugt ist ein Sprühauftrag.

Die aufgebrachte Klebstoffmenge beträgt vorzugsweise 0,5 bis 100 g/m², besonders bevorzugt 2 bis 80 g/m², ganz besonders bevorzugt 10 bis 70 g/m², bezogen auf Klebstoff. Vorzugsweise wird nur die Folie oder nur das Substrat einseitig beschichtet. Es kommt jedoch auch eine Beschichtung von beiden zu verklebenden Einheiten, d.h. von Folie und Substrat in Betracht. Nach der Beschichtung erfolgt üblicherweise eine Trocknung, vorzugsweise bei Raumtemperatur oder bei Temperaturen bis zu 80 °C, um Wasser oder sonstige Lösemittel zu entfernen.

Der Klebstoff kann thermisch aktiviert werden. Die Temperatur in der Klebstoffschicht beträgt vorzugsweise mindestens 30°C oder mindestens 40 °C, z. B. von 30 bis 200 °C, oder von 40 bis 100°C . Ein besonderer Vorteil der Erfindung liegt in einer guten Aktivierbarkeit des Klebstoffs auch bei Temperaturen unterhalb des mit herkömmlichen Klebstoffen verwendeten Temperaturbereiches von 60-70°C, z. B. bei Temperaturen von weniger als 60°C, z. B. maximal 58° C, maximal 55 °C oder maximal 50 °C.

Die Verklebung erfolgt vorzugsweise unter Druck. Dazu können z.B. die zu verklebenden Teile mit einem Druck von mindestens 0,005 oder mindestens 0,01 oder mindestens 0,08 N/mm², z.B. 0,005 bis 5 N/mm² oder 0,01 bis 0,8 N/mm² zusammengepresst werden. Der Anpressdruck kann z. B. durch das Anlegen eines Unterdruckes zwischen Folie und Substrat und/oder durch Luftdruck erzeugt werden.

Besondere Bedeutung hat das erfindungsgemäße Verfahren auch für die Herstellung von Einbauteilen für Fahrzeuge. Besonders bevorzugt ist die Verwendung des erfindungsgemäßen Klebstoffs für die Herstellung von Innenauskleidungsteilen für Automobile. Beispiele für derartige Innenauskleidungsteile sind Türinnenverkleidungen, Schalttafeln, Armaturenbretter, Hutablagen, Fertighimmel, Schiebehimmel, Mittelkonsolen, Handschuhfächer, Sonnenblenden, Säulen, Tür- und Armgriffe, Boden-, Ladeboden- und Kofferraumgruppen sowie Schlafkabinen- und Rückwände der Liefer- und Lastkraftwagen. Hierfür wird insbesondere ein Vakuumtiefziehverfahren oder eine Presskaschierung im Siegelverfahren verwendet. Beim Vakuumtiefziehverfahren wird der Klebstoff auf den Formkörper aufgetragen. Anschließend erfolgt gegebenenfalls ein Ablüften, z.B. bei Raumtemperatur oder im Trockenkanal bei vorzugsweise maximal 40°C. Typischerweise wird die aufzuklebende Folie, z.B. eine Dekorfolie aus luftundurchlässigem Material, in einem Rahmen luftdicht eingespannt. Unterhalb der Folie befindet sich eine Unterform auf die der Formkörper gelegt wird. Unterform und Formkörper sind durchbohrt beziehungsweise luftdurchlässig. Das Gerät ist unterwärts weiter luftdicht abgeschlossen. Beim Absaugen der Luft aus dieser Vorrichtung schmiegt sich nun die Folie unter dem auf seine Oberfläche lastenden atmosphärischen Druck passgenau auf den Formkörper. Die Folie wird vor dem Anlegen des Vakuums, bzw. Unterdruckes, erhitzt. Die Folie ist wegen des zu erzeugenden Vakuums, bzw. Unterdruckes, luftundurchlässig. Beim Presskaschierungsverfahren wird der Klebstoff ebenfalls auf dem Formkörper und gegebenenfalls auf der zur verklebenden Folie aufgetragen, zumindest jedoch auf dem Formkörper. Anschließend erfolgt gegebenenfalls ein Ablüften, typischerweise bei Raumtemperatur oder im Trockenkanal bei vorzugsweise maximal 40°C. Die Verklebung von Formkörpern mit der Folie kann nach Wärmeaktivierung unter Fügen und Pressen erfolgen. Die hier benutzten Folien sind vielfach Kunststoffdekorfolien und weisen eine Oberflächenstruktur auf. Diese Oberflächenstruktur auf der Kunststofffolie kann beispielsweise vor, während oder nach dem Verkleben eingeprägt werden.

Bei dem erfindungsgemäßen Kaschierverfahren zur Herstellung von Verbundfolien wird der oben beschriebene Zweikomponentenklebstoff oder eine entsprechend konfektionierte Zubereitung auf die zu verklebenden Substrate vorzugsweise mit einer Schichtdicke von 0,1 bis 20 g/m², besonders bevorzugt 1 bis 7 g/m² z. B. durch Rakeln, Streichen etc. aufgetragen. Es können übliche Beschichtungsverfahren angewendet werden, z.B. Walzenstreichen, Gegenlaufwalzenstreichen, Gravurwalzenstreichen, Gegenlaufgravurwalzenstreichen, Bürstenstreichen, Stabstreichen, Sprühbeschichten, Luftbürstenbeschichtung, Meniskusbeschichtung, Vorhangbeschichtung oder Tauchbeschichtung. Nach optionaler, kurzer Zeit zur Ablüftung flüchtiger Bestandteile (vorzugsweise nach 1 bis 60 Sekunden) kann das beschichtete Foliensubstrat dann mit einem zweiten Foliensubstrat kaschiert werden, wobei die Temperatur z.B. 20 bis 200 °C, vorzugsweise 20 bis 100 °C und der Druck z. B. 100 bis 3000 kN/m², vorzugsweise 300 bis 2000 kN/m² betragen kann. Als Substrate für die Verbundfolienkaschierung eignen sich besonders z.B. Polymerfolien, insbesondere aus Polyethylen (PE), orientiertem Polypropylen (OPP), ungerecktes Polypropylen (CPP), Polyamid (PA), Polyethylenterephthalat (PET), Polyacetat, Zellophan, mit Metall, z.B. Aluminium, beschichtete (bedampfte) Polymerfolien (kurz : metallisierte Folien) oder Metallfolien, z.B. aus Aluminium. Die genannten Folien können miteinander oder mit einer Folie eines anderen Typs, z. B. Polymerfolien mit Metallfolien, verschiedene Polymerfolien miteinander etc. verklebt werden. Die genannten Folien können beispielsweise auch mit Druckfarben bedruckt sein.

Eine Ausführungsform der Erfindung ist eine Verbundfolie erhältlich nach dem oben genannten Kaschierverfahren, d.h. hergestellt unter Verwendung einer der oben beschriebenen Zweikomponentenklebstoffe. Das Material einer ersten Folie ist vorzugsweise ausgewählt aus OPP, CPP, PE, PET und PA und wobei das Material einer zweiten Folie vorzugsweise ausgewählt ist aus OPP, CPP, PE, PET, PA und Metallfolie. In einer Ausführungsform der Erfindung ist die erste Folie und/oder die zweite Folie auf der jeweiligen Seite, welche mit dem Klebstoff beschichtet wird, bedruckt oder metallisiert. Die Dicke der Substratfolien kann beispielsweise von 5 bis 100 µm, vorzugsweise von 5 bis 40 µm betragen. Bei bevorzugten Verbundfolien ist das Folienmaterial ausgewählt aus der Gruppe bestehend aus Aluminiumfolie, bedruckte Polyesterfolie, unbedruckte Polyesterfolie, bedruckte Polyamidfolie, unbedruckte Polyamidfolie, Polypropylenfolie, Polyethylenfolie und Kombinationen daraus.

Bei der Glanzfolienkaschierung wird ein erstes Substrat mit einem zweiten Substrat laminiert, wobei es sich bei dem ersten Substrat um eine Polymerfolie, vorzugsweise um eine transparente Polymerfolie handelt, und es sich bei dem zweiten Substrat um Papier, Karton oder Pappe handelt, wobei das zweite Substrat vorzugsweise bedruckt ist und wobei die Laminierung vorzugsweise unter Druck und Erwärmen erfolgt. Die Kaschierung erfolgt analog zur Herstellung der Verbundfolien. Vorzugsweise wird das Glanzfolienlaminat hergestellt unter Verwendung des oben beschriebenen Zweikomponentenklebstoffs, wobei das Material einer transparenten Polymerfolie (erstes Substrat) ausgewählt ist aus orientiertem Polypropylen (OPP), ungerecktem Polypropylen (CPP), Polyethylen (PE), Polyamid (PA), Polyethylenterephthalat (PET), Polyacetat und Zellophan und wobei das Material des zweiten Substrats ausgewählt ist aus Papier, Karton und Pappe. Vorzugsweise wird als Polymerfolie für die Glanzfolienkaschierung eine coronabehandelte oPP-Folie verwendet. In einer Ausführungsform der Erfindung ist das zweite Substrat der Glanzfolie auf der Seite, welche mit Klebstoff beschichtet wird, farbig oder bedruckt. Die Dicke der Polymerfolie kann beispielsweise von 5 bis 100 µm, vorzugsweise von 5 bis 40 µm betragen.

Gegenstand der Erfindung ist auch die Verwendung der oben näher beschriebenen härtbaren 2-Komponenten-Zusammemsetzung zum Verkleben in dem oben näher beschriebenen Verfahren, wobei die 2-Komponenten-Zusammensetzung
(a) in einer ersten Komponente mindestens eine Verbindung A enthält mit mindestens einer Cyclothiocarbonat-Einheit mit Fünfringstruktur, wobei drei Glieder des Fünfrings die Struktur -O-C(=O)-S- aufweisen, die beiden übrigen Glieder des Fünfrings C-Atome sind; und
(b) in einer zweiten Komponente mindestens eine Härterverbindung B enthält, welche ausgewählt ist aus Verbindungen, welche mindestens eine funktionelle Gruppe aufweisen, die ausgewählt ist aus primären Amingruppen und sekundären Amingruppen,
wobei optional in der ersten Komponente, in der zweiten Komponente und/oder in einer weiteren Komponente des Klebstoffs mindestens eine Verbindung C enthalten sein kann, welche mindestens eine mit SH-Gruppen reaktive funktionelle Gruppe aufweist.

Gegenstand der Erfindung sind auch verklebte Produkte, hergestellt gemäß dem oben näher beschriebenen Verfahren.

Gegenstand der Erfindung ist auch eine härtbare 2-Komponenten-Klebstoffzusammensetzung, welche
(a) in einer ersten Komponente mindestens eine Verbindung A enthält mit einem Molekulargewicht größer als 1000 g/mol und mit mindestens einer Cyclothiocarbonat-Einheit mit Fünfringstruktur, wobei drei Glieder des Fünfrings die Struktur -O-C(=O)-S- aufweisen, die beiden übrigen Glieder des Fünfrings C-Atome sind; und
(b) in einer zweiten Komponente mindestens eine Härterverbindung B enthält, welche ausgewählt ist aus Verbindungen, welche mindestens eine funktionelle Gruppe aufweisen, die ausgewählt ist aus primären Amingruppen und sekundären Amingruppen, wobei die mindestens eine funktionelle Gruppe auch in maskierter, latent reaktiver Form vorliegen kann; und
(c) optional in der ersten Komponente, in der zweiten Komponente und/oder in einer weiteren Komponente mindestens eine Verbindung C enthalten ist, welche mindestens eine mit SH-Gruppen reaktive funktionelle Gruppe, vorzugsweise mindestens eine Gruppe mit ethylenisch ungesättigter Bindung oder mindestens eine Epoxygruppe enthält, wobei im Falle von Verbindungen mit Epoxygruppen diese in der ersten und/oder in der weiteren Komponente des Klebstoffs enthalten sind;
   wobei die Härterverbindung B in einer Menge eingesetzt wird sodass die Menge n_{B} an funktionellen Gruppen der Härterverbindung B von 80 mol% bis 120 mol% beträgt, bezogen auf die Menge n_{A} an Cyclothiocarbonatgruppen der Verbindung A; und wobei Verbindung C in einer Menge eingesetzt wird, sodass die Menge der mit SH-Gruppen reaktiven Doppelbindungen n_{C3} von 0 bis 110 mol%, vorzugsweise von 90 bis 110 mol% beträgt, bezogen auf die Menge n_{A} an Cyclothiocarbonatgruppen;
   und wobei die Klebstoffzusammensetzung unmittelbar nach Vermischen der Komponenten im noch nicht ausgehärteten Zustand eine Nullviskosität bei 70 °C von vorzugsweise kleiner 300 Pa s aufweist.

Erfindungsgemäße Zusammensetzungen sind insbesondere solche, bei denen die Schälfestigkeit von zwei miteinander mit einer Klebstoffschicht von 3 µm und mit einem Anpressdruck von 3 bar verklebten Polyethylenterephtalatfolien nach 24 Stunden grösser als 1,5 N/15 mm ist.

Die vorliegende Erfindung wird nun anhand der nachfolgenden Beispiele näher erläutert.

### Beispiele

Messung der Nullviskosität:
Die Nullviskosität ist der Grenzwert der Viskositätsfunktion bei unendlich niedrigen Scherraten. Sie wird gemessen mit einem Anton Paar Rheometer MCR 100 (US 200 Auswertesoftware) in Platte/Platte Geometrie. Die Proben werden in oszillatorischer Scherung bei kleiner Scheramplitude von 10% vermessen. Temperatur 23°C (oder wie angegeben), Kreisfrequenzrampe log 100-0,1 1/s, Messspalt 0,5 mm, Auswertung nach Carreau-Gahleitner I, Stempeldurchmesser 25 mm.

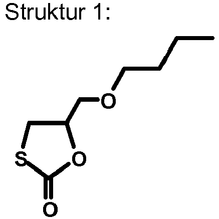

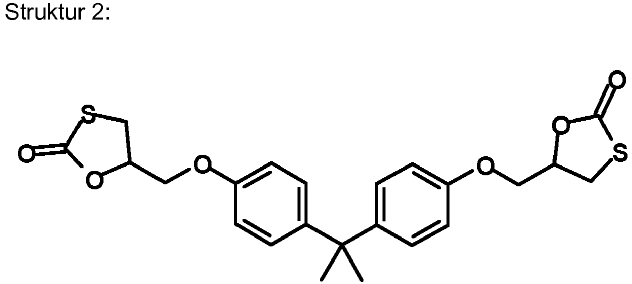

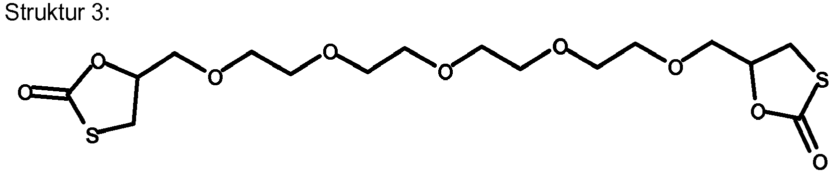

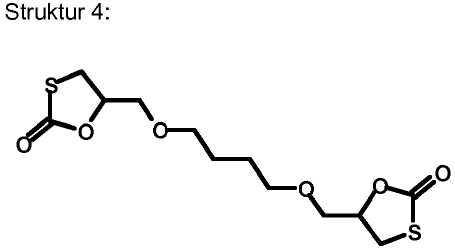

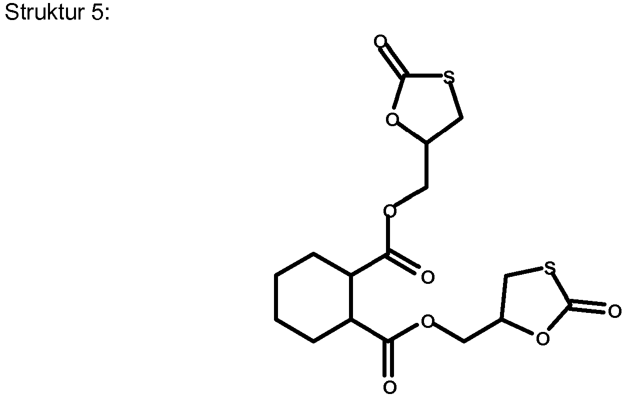

### Beispiel 1

3 g der Verbindung der Struktur 1 wurden mit 1,75 g Trimethylolpropantrimethacrylat (Laromer^{®} TMPTMA, BASF) vermischt, dann 1,6 g Polyethylenimin (Lupasol^{®} FG, BASF) zugemischt und gut verrührt.

Die Mischung wurde auf Edelstahlprüfkörper und auf Aluminiumprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt.

Es wurde auf Edelstahl ein Zugscherfestigkeit von 4,4 MPa gefunden, nach 9 Tagen bei Raumtemperatur 5 MPa und nach 16 Tagen 6 MPa (immer Adhäsionsbruch).

Auf Aluminium wurden nach 48 h 3 MPa gefunden, nach 9 Tagen und nach 16 Tagen jeweils 4 MPa .

### Auftragsmengen bei den Zugscherversuchen sind jeweils 0,2 g/ (2,54 cm)²

### Beispiel 2

2 g der Verbindung der Struktur 1 wurden mit 0,6 g der Verbindung der Struktur 2 und 1,46 g Trimethylolpropantrimethacrylat (Laromer^{®}TMPTMA, BASF) vermischt, dann 1,31 ml 4,9-Di-oxa-dodecan-1,12-diamin (Baxxodur^{®} EC 280, BASF) zupipettiert.

Die Mischung wurde auf Buchenholzprüfkörper (Rocholl GmbH) aufgetragen und mit 2 cm x 4 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt.

### Es wurde eine Zugscherfestigkeit von 1 MPa gefunden, nach 8 Tagen bei Raumtemperatur 2 MPa (jeweils Kohäsionsbruch)

### Beispiel 3

2 g der Verbindung der Struktur 3 wurden mit 1,06 g Trimethylolpropantrimethacrylat (Laromer^{®} TMPTMA, BASF) vermischt, dann 0,968 g Polyethylenimin (Lupasol^{®} FG, BASF) zugemischt und gut verrührt.

Die Mischung wurde auf Edelstahlprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 2,5 MPa (Adhäsions-/Kohäsionsmischbruch) gefunden. Nach 8 Tagen Lagerung bei Raumtemperatur wurde eine Zugscherfestigkeit von 2,7 MPa gefunden.

### Beispiel 4

2 g der Verbindung der Struktur 3 wurden mit 1,06 g Trimethylolpropantrimethacrylat (Laromer^{®} TMPTMA, BASF) vermischt, dann 0,668 g 1,3- Diaminomethylcyclohexan (TCI) zugemischt und gut verrührt.

Die Mischung wurde auf Edelstahlprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 2,1 MPa (A/K Mischbruch) gefunden. Nach 8 Tagen Lagerung bei Raumtemperatur wurde eine Zugscherfestigkeit von 3 MPa gefunden.

### Beispiel 5

2 g der Verbindung der Struktur 1 wurden mit 1,16 g Trimethylolpropantrimethacrylat (Laromer^{®} TMPTMA, BASF) vermischt, dann 0,73 g 1,3-(Bisaminomethyl)-cyclohexan (Sigma-Aldrich) zugemischt und gut verrührt.

### Messung der Schälfestigkeit bei 23 °C

Mit einem Spiralrakel (Erichsen Coater) wurde die Mischung sofort auf eine PET-Folie (Hostaphan^{®} RN 36) gerakelt (Schichtdicke 12 µm) und eine weitere PET-Folie zukaschiert und mit einer 2 kg schweren Rolle zweimal angerollt. Nach 24 h bei Raumtemperatur wurde die Verbundfolie in 15 mm breite Streifen geschnitten. Die beiden Folien des Verbundes wurden in eine Zugmaschine im 90° Winkel geöffnet eingespannt und die Schälfestigkeit mit 100 mm/min geprüft. Es wurde eine Schälfestigkeit von 2 N/15 mm gefunden.

Eine Schälfestigkeit von grösser 1,5 N nach 24 h ist für Anwendungen des Klebstoffs in flexiblen Verpackungen und für Verbundfolienkaschierungen besonders gut geeignet, um industriell verwertbar zu sein.

### Beispiel 6:

2 g der Verbindung der Struktur 4 wurden mit 1,4 g Trimethylolpropantrimethacrylat (Laromer^{®} TMPTMA, BASF) vermischt, mit 0,35 g Kieselgel HDK H13L (Wacker) angedickt; dann 0,883 g 1,3-Diaminomethylcyclohexan (TCI) zugemischt und gut verrührt.

Die Mischung wurde auf Edelstahlprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 11,5 MPa (Adhäsions-Bruch) gefunden.

Die Mischung wurde auch auf Aluminumprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 5 MPa (Adhäsions-Bruch) gefunden.

Die Mischung wurde auch auf ABS-Prüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 2,5 MPa (Adhäsions-Bruch) gefunden.

### Beispiel 7:

2 g der Verbindung der Struktur 5 wurden mit 1,12 g Trimethylolpropantrimethacrylat (Laromer^{®} TMPTMA, BASF) vermischt, mit 0,15 g Kieselgel HDK H13L (Wacker) angedickt; dann 1,09 g 4,7,10-Trioxa-1,13-tridecandiamin (BASF) zugemischt und gut verrührt.

Die Mischung wurde auf Edelstahlprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 5,7 MPa (Adhäsions-Bruch) gefunden.

Die Mischung wurde auch auf Aluminumprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 3,1 MPa (A-Bruch) gefunden. Die Mischung wurde auch auf ABS-Prüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 3,3 MPa (A-Bruch) gefunden.

### Beispiel 8:

1 g der Verbindung der Struktur 4 wurden mit 0,7 g Trimethylolpropantrimethacrylat (Laromer^{®} TMPTMA, BASF) vermischt, mit 0,1 g Kieselgel HDK H13L (Wacker) angedickt; dann 0,62 g 1,4-Bis-(3-aminopropyl)-piperazin (BASF) zugemischt und gut verrührt.

Die Mischung wurde auf Edelstahlprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 9,4 MPa (Adhäsions-Bruch) gefunden.

Die Mischung wurde auch auf Aluminumprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 2,4 MPa (Adhäsions-Bruch) gefunden.

Die Mischung wurde auch auf ABS-Prüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 2,1 MPa (Adhäsions-Bruch) gefunden.

### Beispiel 9:

1 g der Verbindung der Struktur 4 wurden mit 1,59 g Bisphenol-A glycerolat-dimethacrylat (Sigma Aldrich)) vermischt, dann 0,44 g 1,3- Diaminomethylcyclohexan (TCI) zugemischt und gut verrührt.

Die Mischung wird homogen und gut verarbeitbar.

Die Mischung wurde auf Edelstahlprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 6,4 MPa (Adhäsions-Bruch) gefunden. Nach 48 h Aushärtezeit wurden weitere Prüfkörper im Trockenschrank bei 70°C im Wasserdampf in einem geschlossenen Gefäß 7 Tage gelagert. Danach 7 Tage bei Raumtemperatur an der Luft getrocknet und ein Zugscherversuch mit 100 mm/min durchgeführt. Es wurde immer noch eine Zugscherfestigkeit von 2,4 MPa (Adhäsionsbruch) gefunden.

### Beispiel 10:

1 g der Verbindung der Struktur 4 wurden mit 1,15 g Bisphenol -A bis Glycidylether (BADGE) (Sigma Aldrich)) vermischt, dann 0,46 g Jeffamin EDR 148 ( Huntsman) zugemischt und gut verrührt.

Die Mischung wurde auf Edelstahlprüfkörper (Rocholl GmbH) aufgetragen und mit 2,5 cm x 2,5 cm Überlapp verklebt und fixiert. Aushärtung erfolgte über Nacht bei Raumtemperatur. Nach 12 h waren die Prüfkörper handfest verklebt. Nach 48 h wurde ein Zugscherversuch mit 100 mm/min durchgeführt. Es wurde eine Zugscherfestigkeit von 15,3 MPa (Adhäsions-Bruch) gefunden.

Nach 48 h Aushärtezeit wurden weitere Prüfkörper im Trockenschrank bei 70°C im Wasserdampf in einem geschlossenen Gefäß 7 Tage gelagert. Danach 7 Tage bei Raumtemperatur an der Luft getrocknet und ein Zugscherversuch mit 100 mm/min durchgeführt. Es wurde immer noch eine Zugscherfestigkeit von 5,4 MPa (Adhäsions-Bruch) gefunden.

## Patentansprüche

1. Klebeverfahren, wobei zwei Substrate miteinander verklebt werden, indem auf die Oberfläche mindestens eines der Substrate ein härtbarer, noch nicht ausgehärteter, flüssiger 2-Komponentenklebstoff aufgetragen wird, welcher
(a) in einer ersten Komponente mindestens eine Verbindung A enthält mit mindestens einer Cyclothiocarbonat-Einheit mit Fünfringstruktur, wobei drei Glieder des Fünfrings die Struktur -O-C(=O)-S- aufweisen, die beiden übrigen Glieder des Fünfrings C-Atome sind; und
(b) in einer zweiten Komponente mindestens eine Härterverbindung B enthält, welche ausgewählt ist aus Verbindungen, welche mindestens eine funktionelle Gruppe aufweisen, die ausgewählt ist aus primären Amingruppen und sekundären Amingruppen, wobei die mindestens eine funktionelle Gruppe auch in maskierter, latent reaktiver Form vorliegen kann,
und wobei optional in der ersten Komponente, in der zweiten Komponente und/ oder in einer weiteren Komponente des Klebstoffs mindestens eine Verbindung C enthalten sein kann, welche mindestens eine mit SH-Gruppen reaktive funktionelle Gruppe aufweist.

2. Klebeverfahren nach Anspruch 1 **dadurch gekennzeichnet, dass** die mit SH-Gruppen reaktive funktionelle Gruppe ausgewählt ist aus Gruppen mit ethylenisch ungesättigter Bindung und Epoxygruppen und mindestens zwei der Verbindungen A, B und C multifunktionell sind.

3. Klebeverfahren nach Anspruch 1 oder 2 **dadurch gekennzeichnet, dass**
(a) Verbindung A eine Anzahl von n_{A} der Cyclothiocarbonat-Einheiten, wobei n_{A} eine ganze Zahl größer oder gleich 1 ist;
(b) Verbindung B eine Anzahl von n_{B} der funktionellen Gruppen und eine Anzahl von n_{C2} an mit SH-Gruppen reaktiven ethylenisch ungesättigten Bindungen aufweist, wobei n_{B} eine ganze Zahl größer oder gleich 1 ist und n_{C2} eine ganze Zahl größer oder gleich 0 ist; und
(c) optional in der ersten Komponente, in der zweiten Komponente und/oder in einer weiteren Komponente mindestens eine Verbindung C enthalten ist, welche eine Anzahl n_{C3} an mit SH-Gruppen reaktiven funktionellen Gruppen enthält und n_{C3} eine ganze Zahl größer oder gleich 1 ist;
mit der Massgabe, dass mindestens eine Zahl von n_{A} und der Summe von n_{B} + n_{C2} größer oder gleich 2 ist;
und dass wenn n_{C2} Null ist, dann entweder n_{C3} größer oder gleich 2 ist oder sowohl n_{A} als auch n_{B} größer oder gleich 2 sind oder sowohl n_{A} als auch n_{B} als auch n_{C3} größer oder gleich 2 sind.

4. Verfahren nach einem der vorhergehenden Ansprüche, **dadurch gekennzeichnet, dass** Verbindung A die Formel (I) aufweist 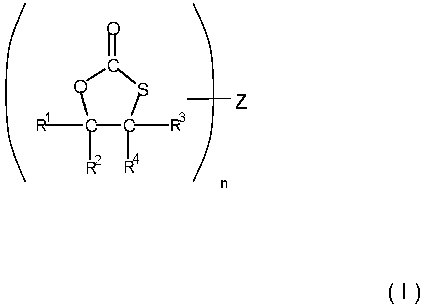 wobei R¹ bis R⁴ unabhängig voneinander Wasserstoff oder eine organische Gruppe mit vorzugsweise bis zu 50 C-Atomen bedeuten, wobei alternativ R², R⁴ und die beiden C-Atome der Monothiocarbonatgruppe zusammen einen fünf- bis zehngliedrigen Ring bilden können, und wobei eine der Gruppen R¹ bis R⁴ eine Verbindungsgruppe zu Z ist, wobei die Verbindungsgruppe auch eine chemische Bindung sein kann, n eine ganze Zahl von größer oder gleich 1 bedeutet und Z für Wasserstoff oder für eine n-valente organische Gruppe steht.

5. Verfahren nach Anspruch 4, **dadurch gekennzeichnet dass** drei der Gruppen R¹ bis R⁴ in Formel I Wasserstoff sind und die übrige Gruppe von R¹ bis R⁴ die Verbindungsgruppe zu Z ist, wobei die Verbindungsgruppe auch eine chemische Bindung sein kann.

6. Verfahren nach einem der Ansprüche 4 bis 5, **dadurch gekennzeichnet dass** die Verbindungsgruppe zu Z eine Einfachbindung ist oder eine Ethergruppe -CH₂-O- oder eine Estergruppe -CH₂-O-C(=O)-.

7. Verfahren nach einem der Ansprüche 4 bis 6, **dadurch gekennzeichnet dass** Z eine n-valente organische Gruppe ist mit bis zu 50 C-Atomen, die O-Atome enthalten kann und n eine Zahl von 2 bis 5, vorzugsweise 2 ist.

8. Verfahren nach einem der vorhergehenden Ansprüche, **dadurch gekennzeichnet dass** Verbindung A die Formel (II) aufweist 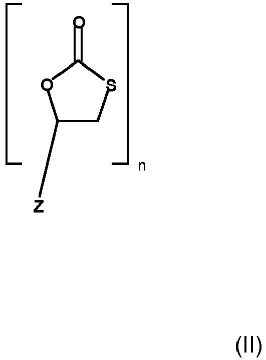 wobei n eine Zahl größer oder gleich 1, vorzugsweise größer oder gleich 2 ist, und Z für Wasserstoff oder für eine n-valente organische Gruppe steht, vorzugsweise für Alkyl, Aryl, Alkenyl, oder Aralkyl, wobei die Gruppe Z substituiert oder unsubstituiert sein kann und wobei die Gruppe Z unterbrochen sein kann durch O, Halogen, S, C=O, O-C=O, O-(C=O)-O oder (C=O)-NR, wobei R Wasserstoff oder eine organische Gruppe bedeutet.

9. Verfahren nach einem der Ansprüche 4 bis 8, **dadurch gekennzeichnet dass** Z eine Alkoxylengruppe oder eine Polyalkoxylengruppe ist der Formel G1:
-(V-O-)mV (G1)
wobei V eine C2- bis C20 Alkylengruppe bedeutet und m eine Zahl größer oder gleich 1 ist.

10. Verfahren nach einem der Ansprüche 4 bis 9, **dadurch gekennzeichnet dass** Z eine Gruppe ist der Formel G2: 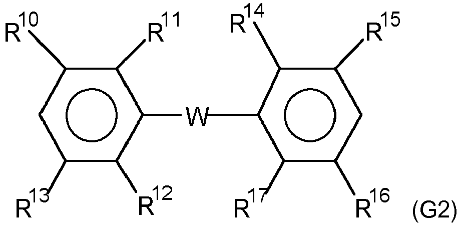 wobei W eine bi-valente organische Gruppe bedeutet mit bis zu 10 C-Atomen und R¹⁰ bis R¹⁷ unabhängig voneinander H oder eine C1- bis C4-Alkylgruppe bedeuten.

11. Verfahren nach einem der Ansprüche 1 bis 6, **dadurch gekennzeichnet dass** Verbindung A ausgewählt ist aus der Gruppe bestehend aus
Verbindungen der Formel (III) 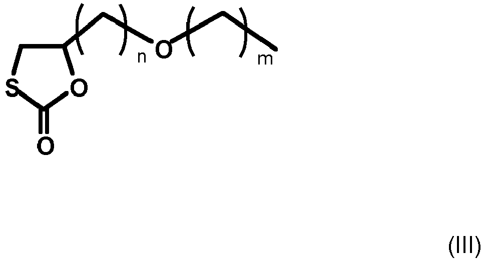 wobei n eine Zahl von 1 bis 10, vorzugsweise 1 ist und m eine Zahl von 0 bis 9, vorzugsweise1 bis 5 ist; und
Verbindungen der Formel (IV) 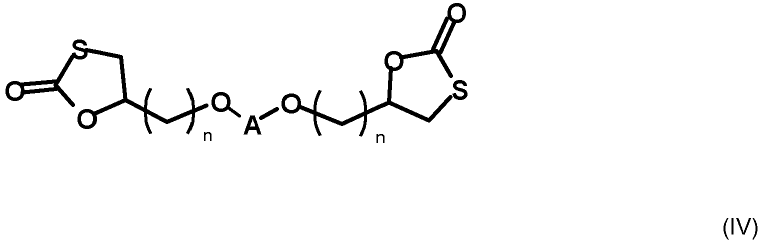 wobei n eine Zahl von 1 bis 10, vorzugsweise 1 ist und A ein Gruppe ist ausgewählt aus - Ph-CRₐR_{b}-Ph- und -(CH₂-CH₂-O)ₘ- CH₂-CH₂-,
wobei Rₐ und R_{b} unabhängig voneinander für H oder C1 bis C4-Alkyl, vorzugsweise für Methyl stehen; und m eine Zahl von 0 bis 10, vorzugsweise 1 bis 5 ist.

12. Verfahren nach einem der vorhergehenden Ansprüche, **dadurch gekennzeichnet dass** Verbindung B eine Verbindung ist ausgewählt aus Polyaminen mit mindestens zwei primären oder sekundären Amingruppen.

13. Verfahren nach einem der vorhergehenden Ansprüche, **dadurch gekennzeichnet dass** Verbindung B eine Verbindung ist mit mindestens zwei primären Amingruppen.

14. Verfahren nach einem der Ansprüche 2 bis 13, **dadurch gekennzeichnet dass** die mit SH-Gruppen reaktiven funktionellen Gruppen ausgewählt sind aus ethylenisch ungesättigten Bindungen, welche ausgewählt sind aus (meth)acrylischen, allylischen und vinylischen C-C-Doppelbindungen und C-C-Dreifachbindungen und aus Epoxygruppen.

15. Verfahren nach einem der vorhergehenden Ansprüche, **dadurch gekennzeichnet dass** in mindestens einer Klebstoffkomponente mindestens eine Verbindung C enthalten ist, die ausgewählt ist aus Trimethylolpropantri(meth)acrylat und Alkandioldi(meth)acrylaten von C2- bis C8-Alkandiolen.

16. Verfahren nach einem der vorhergehenden Ansprüche, **dadurch gekennzeichnet dass** Härterverbindung B in einer Menge eingesetzt wird, sodass die Menge n_{B} an funktionellen Gruppen der Härterverbindung B von 80 mol% bis 120 mol% beträgt, bezogen auf die Menge n_{A} an Cyclothiocarbonatgruppen der Verbindung A; und dass Verbindung C in einer Menge eingesetzt wird, sodass die Menge der reaktiven Doppelbindungen n_{C3} von 0 bis 120 mol%, vorzugsweise von 90 bis 110 mol% beträgt, bezogen auf die Menge n_{A} an Cyclothiocarbonatgruppen.

17. Verfahren nach einem der vorhergehenden Ansprüche, **dadurch gekennzeichnet dass** der Zweikomponentenklebstoff mit einer Auftragsmenge von 0,5 bis 1000 g/m², bevorzugt 1 - 100 g/m² besonders bevorzugt von 10 bis 70 g/m² aufgetragen wird.

18. Verfahren nach einem der vorhergehenden Ansprüche, **dadurch gekennzeichnet dass** die zu verklebenden Substrate gleich oder voneinander verschieden sind und ausgewählt sind aus Metall, Holz, Glas, Kunststoffformkörpern, Kunststofffolien, Papier und Pappe.

19. Klebeverfahren nach einem der vorhergehenden Ansprüche, wobei es sich um ein Kaschierverfahren zur Herstellung von kaschierten Gegenständen, ausgewählt aus Glanzfolien, Verbundfolien und kaschierten Formkörpern, handelt, wobei man
a) ein erstes Substrat in Form einer ersten Folie zur Verfügung stellt,
b) ein zweites Substrat zur Verfügung stellt, ausgewählt aus Papier, einer zweiten Folie, die zur ersten Folie gleich oder verschieden sein kann, und Formkörpern,
c) einen Zweikomponentenklebstoff gemäß einem der Ansprüche 1 bis 16 zur Verfügung stellt und
d) den Zweikomponentenklebstoff auf das erste Substrat und/oder auf das zweite Substrat aufträgt, optional trocknen lässt und das erste Substrat auf das zweite Substrat aufkaschiert.

20. Klebeverfahren nach einem der vorhergehenden Ansprüche, **dadurch gekennzeichnet, dass** der Klebstoff mindestens einen Katalysator enthält für die Katalysierung der Reaktion der Cyclocarbonatgruppen der Verbindung A mit den funktionellen Gruppen der Härterverbindung B oder dass der Klebstoff mindestens einen Katalysator oder Initiator enthält für die Katalysierung oder Initiierung der Reaktion von SH-Gruppen miteinander oder der Reaktion von SH-Gruppen mit ethylenisch ungesättigten Doppelbindungen.

21. Klebeverfahren nach einem der vorhergehenden Ansprüche, wobei die Verklebung bei Temperaturen kleiner oder gleich 30°C, vorzugsweise ohne Erwärmung erfolgt.

22. Verwendung einer härtbaren 2-Komponenten-Zusammensetzung zum Verkleben in einem Verfahren gemäß einem der Ansprüche 1 bis 21 wobei die 2-Komponenten-Zusammensetzung
(a) in einer ersten Komponente mindestens eine Verbindung A enthält mit mindestens einer Cyclothiocarbonat-Einheit mit Fünfringstruktur, wobei drei Glieder des Fünfrings die Struktur -O-C(=O)-S- aufweisen, die beiden übrigen Glieder des Fünfrings C-Atome sind; und
(b) in einer zweiten Komponente mindestens eine Härterverbindung B enthält, welche ausgewählt ist aus Verbindungen, welche mindestens eine funktionelle Gruppe aufweisen, die ausgewählt ist aus primären Amingruppen und sekundären Amingruppen,
wobei optional in der ersten Komponente, in der zweiten Komponente und/ oder in einer weiteren Komponente des Klebstoffs mindestens eine Verbindung C enthalten sein kann, welche mindestens eine mit SH-Gruppen reaktive funktionelle Gruppe aufweist.

23. Verklebtes Produkt, hergestellt gemäß dem Verfahren gemäß einem der Ansprüche 1 bis 21.

24. Härtbare 2-Komponenten-Klebstoffzusammensetzung, welche
(a) in einer ersten Komponente mindestens eine Verbindung A enthält mit einem Molekulargewicht größer als 1000 g/mol und mit mindestens einer Cyclothiocarbonat-Einheit mit Fünfringstruktur, wobei drei Glieder des Fünfrings die Struktur -O-C(=O)-S- aufweisen, die beiden übrigen Glieder des Fünfrings C-Atome sind; und
(b) in einer zweiten Komponente mindestens eine Härterverbindung B enthält, welche ausgewählt ist aus Verbindungen, welche mindestens eine funktionelle Gruppe aufweisen, die ausgewählt ist aus primären Amingruppen und sekundären Amingruppen, wobei die mindestens eine funktionelle Gruppe auch in maskierter, latent reaktiver Form vorliegen kann; und
(c) optional in der ersten Komponente, in der zweiten Komponente und/oder in einer weiteren Komponente mindestens eine Verbindung C enthalten ist, welche mindestens eine mit SH-Gruppen reaktive funktionelle Gruppe, vorzugsweise mindestens eine Gruppe mit ethylenisch ungesättigter Bindung oder mindestens eine Epoxygruppe, enthält, wobei im Falle von Verbindungen mit Epoxygruppen diese in der ersten und/oder in der weiteren Komponente des Klebstoffs enthalten sind;
wobei die Härterverbindung B in einer Menge eingesetzt wird sodass die Menge n_{B} an funktionellen Gruppen der Härterverbindung B von 80 mol% bis 120 mol% beträgt, bezogen auf die Menge n_{A} an Cyclothiocarbonatgruppen der Verbindung A; und wobei Verbindung C in einer Menge eingesetzt wird, sodass die Menge der mit SH-Gruppen reaktiven Doppelbindungen n_{C3} von 0 bis 110 mol%, vorzugsweise von 90 bis 110 mol% beträgt, bezogen auf die Menge n_{A} an Cyclothiocarbonatgruppen;
und wobei die Klebstoffzusammensetzung unmittelbar nach Vermischen der Komponenten im noch nicht ausgehärteten Zustand vorzugsweise eine Nullviskosität bei 70 °C von kleiner 300 Pa s aufweist.

25. Härtbare 2-Komponenten-Klebstoffzusammensetzung, nach dem vorhergehenden Anspruch, **dadurch gekennzeichnet, dass** die Schälfestigkeit von zwei miteinander mit einer Klebstoffschicht von 3 µm und mit einem Anpressdruck von 3 bar verklebten Polyethylenterephtalatfolien nach 24 Stunden grösser als 1,5 N/15 mm ist.

## Claims

1. A bonding process in which two substrates are bonded together by
applying to the surface of at least one of the substrates a curable, not yet cured, liquid 2-component adhesive that
(a) in a first component comprises at least one compound A having at least one cyclic thiocarbonate unit having a five-membered ring structure in which three members of the five-membered ring have the structure -O-C(=O)-S-, the two remaining members of the five-membered ring being carbon atoms; and
(b) in a second component comprises at least one curing compound B selected from compounds having at least one functional group selected from primary amine groups and secondary amine groups, wherein the at least one functional group may also be present in masked, latently reactive form,
and wherein the first component, the second component, and/or a further component of the adhesive may optionally comprise at least one compound C that has at least one functional group reactive toward SH groups.

2. The bonding process according to claim 1, wherein the functional group reactive toward SH groups is selected from groups having ethylenically unsaturated bond and epoxy groups and at least two of the compounds A, B, and C are polyfunctional.

3. The bonding process according to claim 1 or 2, wherein
(a) compound A a number n_{A} of cyclic thiocarbonate units, n_{A} being an integer greater than or equal to 1;
(b) compound B has a number n_{B} of functional groups and a number n_{C2} of ethylenically unsaturated bonds reactive toward SH groups, n_{B} being an integer greater than or equal to 1 and n_{C2} being an integer greater than or equal to 0; and
(c) the first component, the second component, and/or a further component optionally comprises at least one compound C that contains a number n_{C3} of functional groups reactive toward SH groups, n_{C3} being an integer greater than or equal to 1;
with the proviso that at least one number out of n_{A} and the sum of n_{B} + n_{C2} is greater than or equal to 2;
and that when n_{C2} is zero, then either n_{C3} is greater than or equal to 2, or both n_{A} and n_{B} are greater than or equal to 2, or both n_{A} and n_{B} and n_{C3} are greater than or equal to 2.

4. The process according to any of the preceding claims, wherein compound A has the formula (I) 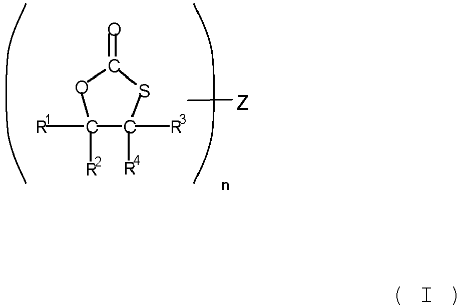 where R¹ to R⁴ are independently hydrogen or an organic group having preferably up to 50 carbon atoms; alternatively R², R⁴, and the two carbon atoms of the monothiocarbonate group may together form a five- to ten-membered ring, one of the groups R¹ to R⁴ being a linking group to Z, wherein the linking group may also be a chemical bond, n is an integer greater than or equal to 1, and Z is hydrogen or an n-valent organic group.

5. The process according to claim 4, wherein three of the groups R¹ to R⁴ in formula I are hydrogen and the remaining group from R¹ to R⁴ is the linking group to Z, wherein the linking group may also be a chemical bond.

6. The process according to either of claims 4 or 5, wherein the linking group to Z is a single bond or an ether group -CH₂-O- or an ester group -CH₂-O-C(=O)-.

7. The process according to any of claims 4 to 6, wherein Z is an n-valent organic group having up to 50 carbon atoms, which may contain oxygen atoms, and n is a number from 2 to 5, preferably 2.

8. The process according to any of the preceding claims, wherein compound A has the formula (II) 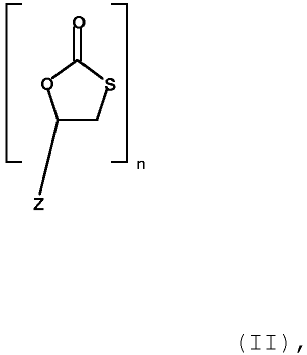 where n is a number greater than or equal to 1, preferably greater than or equal to 2, and Z is hydrogen or an n-valent organic group, preferably alkyl, aryl, alkenyl, or aralkyl, in which the group Z may be substituted or unsubstituted and in which the group Z may be interrupted by O, halogen, S, C=O, O-C=O, O-(C=O)-O or (C=O)-NR, where R is hydrogen or an organic group.

9. The process according to any of claims 4 to 8, wherein Z is an alkoxylene group or a polyalkoxylene group of the formula G1:
-(V-O-)ₘV (G1),
where V is a C2 to C20 alkylene group and m is a number greater than or equal to 1.

10. The process according to any of claims 4 to 9, wherein Z is a group of the formula G2: 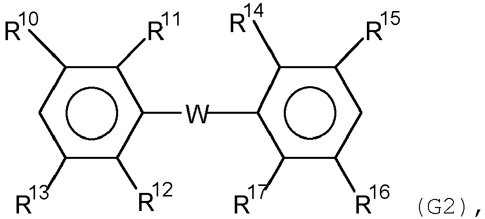 where W is a divalent organic group having up to 10 carbon atoms and R¹⁰ to R¹⁷ are independently H or a C1 to C4 alkyl group.

11. The process according to any of claims 1 to 6, wherein compound A is selected from the group consisting of compounds of the formula (III) 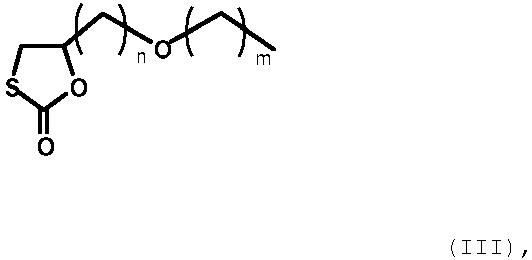 where n is a number from 1 to 10, preferably 1, and m is a number from 0 to 9, preferably 1 to 5; and compounds of the formula (IV) 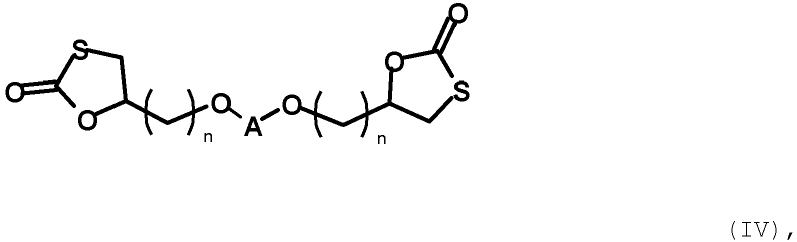 where n is a number from 1 to 10, preferably 1, and A is a group selected from -Ph-CRₐR_{b}-Ph- and -(CH₂-CH₂-O)ₘ-CH₂-CH₂-,
where Rₐ and R_{b} are independently H or C1 to C4 alkyl, preferably methyl; and m is a number from 0 to 10, preferably 1 to 5.

12. The process according to any of the preceding claims, wherein compound B is a compound selected from polyamines having at least two primary or secondary amine groups.

13. The process according to any of the preceding claims, wherein compound B is a compound having at least two primary amine groups.

14. The process according to any of claims 2 to 13, wherein the functional groups reactive toward SH groups are selected from ethylenically unsaturated bonds, which are selected from (meth)acrylic, allylic, and vinylic C-C double bonds and C-C triple bonds and from epoxy groups.

15. The process according to any of the preceding claims, wherein at least one of the components of the adhesive comprises at least one compound C selected from trimethylolpropane tri(meth)acrylate and alkanediol di(meth)acrylates of C2 to C8 alkanediols.

16. The process according to any of the preceding claims, wherein the curing compound B is used in an amount such that the amount n_{B} of functional groups in curing compound B is from 80 mol% to 120 mol%, based on the amount n_{A} of cyclic thiocarbonate groups in compound A; and that compound C is used in an amount such that the amount of reactive double bonds n_{C3} is from 0 to 120 mol%, preferably from 90 to 110 mol%, based on the amount n_{A} of cyclic thiocarbonate groups.

17. The process according to any of the preceding claims, wherein the two-component adhesive is applied in an applied amount from 0.5 to 1000 g/m², preferably 1-100 g/m², more preferably from 10 to 70 g/m².

18. The process according to any of the preceding claims, wherein the substrates to be bonded are the same as or different from one another and are selected from metal, wood, glass, plastic shaped bodies, plastic films, paper, and paperboard.

19. The bonding process according to any of the preceding claims, wherein it is a lamination process for producing laminated articles selected from glossy films, composite films, and laminated shaped bodies, wherein
a) a first substrate in the form of a first film is provided,
b) a second substrate is provided, selected from paper, a second film that may be the same as or different from the first film, and shaped bodies,
c) a two-component adhesive according to any of claims 1 to 16 is provided, and
d) the two-component adhesive is applied onto the first substrate and/or onto the second substrate, optionally being allowed to dry, and the first substrate is laminated onto the second substrate.

20. The bonding process according to any of the preceding claims, wherein the adhesive comprises at least one catalyst for catalyzing the reaction of the cyclic carbonate groups in compound A with the functional groups in curing compound B or wherein the adhesive comprises at least one catalyst or initiator for catalyzing or initiating the reaction of SH groups with one another or the reaction of SH groups with ethylenically unsaturated double bonds.

21. The bonding process according to any of the preceding claims, wherein bonding takes place at temperatures of less than or equal to 30°C, preferably without heating.

22. The use of a curable 2-component composition for bonding in a process according to any of claims 1 to 21, wherein the 2-component composition
(a) in a first component comprises at least one compound A having at least one cyclic thiocarbonate unit having a five-membered ring structure in which three members of the five-membered ring have the structure -O-C(=O)-S-, the two remaining members of the five-membered ring being carbon atoms; and
(b) in a second component comprises at least one curing compound B selected from compounds having at least one functional group selected from primary amine groups and secondary amine groups,
wherein the first component, the second component, and/or a further component of the adhesive may optionally comprise at least one compound C that has at least one functional group reactive toward SH groups.

23. A bonded product produced according to the process according to any of claims 1 to 21.

24. A curable 2-component adhesive composition that
(a) in a first component comprises at least one compound A having a molecular weight greater than 1000 g/mol and having at least one cyclic thiocarbonate unit having a five-membered ring structure in which three members of the five-membered ring have the structure -O-C(=O)-S-, the two remaining members of the five-membered ring being carbon atoms; and
(b) in a second component comprises at least one curing compound B selected from compounds having at least one functional group selected from primary amine groups and secondary amine groups, wherein the at least one functional group may also be present in masked, latently reactive form; and
(c) optionally in the first component, in the second component, and/or in a further component at least one compound C is present that comprises at least one functional group reactive toward SH groups, preferably at least one group having an ethylenically unsaturated bond or at least one epoxy group; in the case of compounds having epoxy groups, these are present in the first and/or in the further component of the adhesive;
wherein the curing compound B is used in an amount such that the amount n_{B} of functional groups in curing compound B is from 80 mol% to 120 mol%, based on the amount n_{A} of cyclic thiocarbonate groups in compound A; and wherein compound C is used in an amount such that the amount of double bonds n_{C3} reactive toward SH groups is from 0 to 110 mol%, preferably from 90 to 110 mol%, based on the amount n_{A} of cyclic thiocarbonate groups;
and wherein the adhesive composition in the not yet cured state immediately after the components have been mixed preferably has a zero-shear viscosity at 70°C of less than 300 Pa s.

25. A curable 2-component adhesive composition according to the preceding claim, wherein the peel strength after 24 hours of two polyethylene terephthalate films bonded together with an adhesive layer of 3 µm and with a contact pressure of 3 bar is greater than 1.5 N/15 mm.

## Revendications

1. Procédé de collage, deux substrats étant collés l'un avec l'autre par le fait qu'un adhésif à 2 composants durcissable, pas encore durci, liquide est appliqué sur la surface d'au moins un des substrats, lequel
(a) contient dans un premier composant au moins un composé A comportant au moins un motif de cyclothiocarbonate doté d'une structure cyclique à cinq chaînons, trois chaînons du cycle à cinq chaînons présentant la structure -O-C(=O)-S-, les deux autres chaînons du cycle à cinq chaînons étant des atomes de C ; et
(b) contient dans un deuxième composant au moins un composé durcisseur B qui est choisi parmi des composés qui présentent au moins un groupe fonctionnel qui est choisi parmi des groupes amine primaire et des groupes amine secondaire, l'au moins un groupe fonctionnel pouvant être présent également sous forme masquée, à réactivité latente,
et, éventuellement dans le premier composant, dans le deuxième composant et/ou dans un composant supplémentaire de l'adhésif, au moins un composé C pouvant être contenu, qui présente au moins un groupe fonctionnel réactif envers des groupes SH.

2. Procédé de collage selon la revendication 1, **caractérisé en ce que** le groupe fonctionnel réactif envers des groupes SH est choisi parmi des groupes comportant une liaison éthyléniquement insaturée et des groupes époxy et au moins deux des composés A, B et C étant polyfonctionnels.

3. Procédé de collage selon la revendication 1 ou 2 **caractérisé en ce que**
(a) le composé A présente un nombre n_{A} de motifs de cyclothiocarbonate, n_{A} étant un nombre entier supérieur ou égal à 1 ;
(b) le composé B présente un nombre n_{B} de groupes fonctionnels et un nombre n_{C2} de liaisons éthyléniquement insaturées réactives envers des groupes SH, n_{B} étant un nombre entier supérieur ou égal à 1 et n_{C2} étant un nombre entier supérieur ou égal à 0 ; et
(c) éventuellement au moins un composé C est contenu, dans le premier composant, dans le deuxième composant et/ou dans un composant supplémentaire, lequel contient un nombre n_{C3} de groupes fonctionnels réactifs envers des groupes SH et n_{C3} étant un nombre entier supérieur ou égal à 1 ;
à la condition qu'au moins un nombre de n_{A} et de la somme de n_{B} + n_{C2} soit supérieur ou égal à 2 ;
et **en ce que** lorsque n_{C2} est zéro, alors soit n_{C3} est supérieur ou égal à 2, soit aussi bien n_{A} que n_{B} sont supérieurs ou égaux à 2 ou aussi bien n_{A} que n_{B} et n_{C3} sont supérieurs ou égaux à 2.

4. Procédé selon l'une quelconque des revendications précédentes, **caractérisé en ce que** le composé A présente la formule (I) 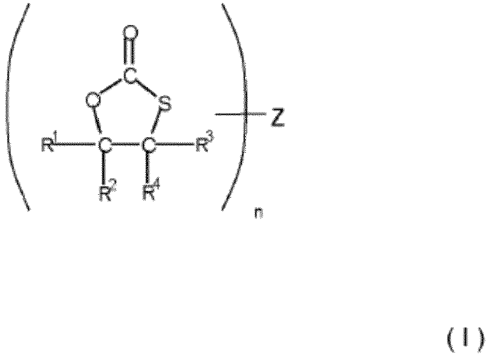 R¹ à R⁴ signifiant indépendamment les uns des autres hydrogène ou un groupe organique comportant de préférence jusqu'à 50 atomes de C, en variante, R², R⁴ et les deux atomes de C du groupe monothiocarbonate pouvant former ensemble un cycle comprenant cinq à dix chaînons, et l'un des groupes R¹ à R⁴ étant un groupe de liaison à Z, le groupe de liaison pouvant également être une liaison chimique, n signifiant un nombre entier supérieur ou égal à 1 et Z représentant hydrogène ou un groupe organique n-valent.

5. Procédé selon la revendication 4, **caractérisé en ce que** trois des groupes R¹ à R⁴ dans la formule I sont hydrogène et l'autre groupe de R¹ à R⁴ est le groupe de liaison à Z, le groupe de liaison pouvant également être une liaison chimique.

6. Procédé selon l'une quelconque des revendications 4 et 5, **caractérisé en ce que** le groupe de liaison à Z est une simple liaison ou un groupe éther -CH₂-O- ou un groupe ester -CH₂-O-C(=O)-.

7. Procédé selon l'une quelconque des revendications 4 à 6, **caractérisé en ce que** Z est un groupe organique n-valent comportant jusqu'à 50 atomes de C qui peut contenir des atomes de O et n est un nombre de 2 à 5, de préférence 2.

8. Procédé selon l'une quelconque des revendications précédentes, **caractérisé en ce que** le composé A présente la formule (II) 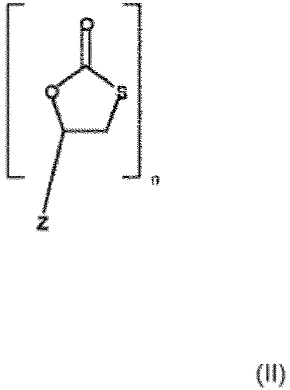 n étant un nombre supérieur ou égal à 1, de préférence supérieur ou égal à 2, et Z représentant hydrogène ou un groupe organique n-valent, de préférence représentant alkyle, aryle, alcényle ou aralkyle, le groupe Z pouvant être substitué ou non substitué et le groupe Z pouvant être interrompu par O, halogène, S, C=O, O-C=O, O-(C=O)-O ou (C=O)-NR, R signifiant hydrogène ou un groupe organique.

9. Procédé selon l'une quelconque des revendications 4 à 8, **caractérisé en ce que** Z est un groupe alcoxylène ou un groupe polyalcoxylène de formule G1 :
**-(V-O-)ₘV** **(G1)**
V signifiant un groupe alkylène en C2 à C20 et m étant un nombre supérieur ou égal à 1.

10. Procédé selon l'une quelconque des revendications 4 à 9, **caractérisé en ce que** Z est un groupe de formule G2 : 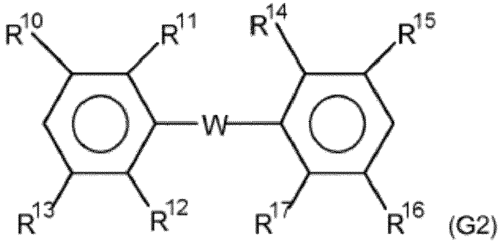 W signifiant un groupe organique bivalent comportant jusqu'à 10 atomes de C et R¹⁰ à R¹⁷ signifiant indépendamment l'un de l'autre H ou un groupe alkyle en C1 à C4.

11. Procédé selon l'une quelconque des revendications 1 à 6, **caractérisé en ce que** le composé A est choisi dans le groupe constitué par
des composés de formule (III) 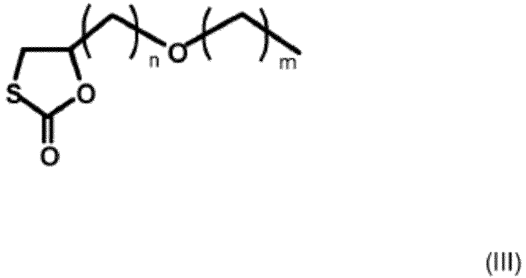 n étant un nombre de 1 à 10, de préférence 1 et m étant un nombre de 0 à 9, de préférence de 1 à 5 ; et
des composés de formule (IV) 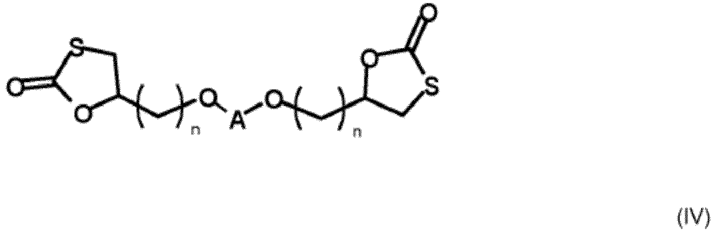 n étant un nombre de 1 à 10, de préférence 1 et A étant un groupe choisi parmi Ph-CRₐR_{b}-Ph- et -(CH₂CH₂-O)ₘ-CH₂-CH₂-.
Rₐ et R_{b} représentant indépendamment l'un de l'autre H ou alkyle en C1 à C4, de préférence représentant méthyle ; et m étant un nombre de 0 à 10, de préférence de 1 à 5.

12. Procédé selon l'une quelconque des revendications précédentes, **caractérisé en ce que** le composé B est un composé choisi parmi des polyamines comportant au moins deux groupes amine primaire ou secondaire.

13. Procédé selon l'une quelconque des revendications précédentes, **caractérisé en ce que** le composé B est un composé comportant au moins deux groupes amine primaire.

14. Procédé selon l'une quelconque des revendications 2 à 13, **caractérisé en ce que** les groupes fonctionnels réactifs envers des groupes SH sont choisis parmi des liaisons éthyléniquement insaturées, qui sont choisies parmi des doubles liaisons C-C (méth)acryliques, allyliques et vinyliques et des triples liaisons C-C et parmi des groupes époxy.

15. Procédé selon l'une quelconque des revendications précédentes, **caractérisé en ce que** dans au moins un composant d'adhésif au moins un composé C est contenu qui est choisi parmi un tri(méth)acrylate de triméthylolpropane et des di(méth)acrylates d'alcanediol d'alcanediols en C2 à C8.

16. Procédé selon l'une quelconque des revendications précédentes, **caractérisé en ce que** le composé durcisseur B est utilisé en une quantité telle que la quantité n_{B} de groupes fonctionnels du composé durcisseur B soit de 80 % en moles à 120 % en moles, par rapport à la quantité n_{A} de groupes cyclothiocarbonate du composé A ; et **en ce que** le composé C est utilisé en une quantité telle que la quantité des doubles liaisons réactives n_{C3} soit de 0 à 120 % en moles, de préférence de 90 à 110 % en moles, par rapport à la quantité n_{A} de groupes cyclothiocarbonate.

17. Procédé selon l'une quelconque des revendications précédentes, **caractérisé en ce que** l'adhésif à deux composants est appliqué avec une quantité d'application de 0,5 à 1 000 g/m², préférablement de 1 à 100 g/m², particulièrement préférablement de 10 à 70 g/m².

18. Procédé selon l'une quelconque des revendications précédentes, **caractérisé en ce que** les substrats à coller sont identiques ou différents l'un de l'autre et choisis parmi du métal, du bois, du verre, des corps moulés de matière plastique, des feuilles de matière plastique, du papier et du carton.

19. Procédé de collage selon l'une quelconque des revendications précédentes, qui est un procédé de contrecollage pour la préparation d'objets contrecollés, choisis parmi des feuilles brillantes, des feuilles composites et des corps moulés contrecollés, dans lequel
a) on met à disposition un premier substrat sous forme d'une première feuille,
b) on met à disposition un deuxième substrat, choisi parmi du papier, une deuxième feuille, qui peut être identique ou différente de la première feuille, et des corps moulés,
c) on met à disposition un adhésif à deux composants selon l'une quelconque des revendications 1 à 16 et
d) on applique l'adhésif à deux composants sur le premier substrat et/ou sur le deuxième substrat, on laisse éventuellement sécher et on contrecolle le premier substrat sur le deuxième substrat.

20. Procédé de collage selon l'une quelconque des revendications précédentes, **caractérisé en ce que** l'adhésif contient au moins un catalyseur pour la catalyse de la réaction des groupes cyclocarbonate du composé A avec les groupes fonctionnels du composé durcisseur B ou **en ce que** l'adhésif contient au moins un catalyseur ou initiateur pour la catalyse ou l'initiation de la réaction de groupes SH les uns avec les autres ou de la réaction de groupes SH avec des doubles liaisons éthyléniquement insaturées.

21. Procédé de collage selon l'une quelconque des revendications précédentes, le collage étant réalisé à des températures inférieures ou égales à 30 °C, de préférence sans chauffage.

22. Utilisation d'une composition durcissable à 2 composants pour le collage dans un procédé selon l'une quelconque des revendications 1 à 21, la composition à 2 composants
(a) contenant dans un premier composant au moins un composé A comportant au moins un motif de cyclothiocarbonate doté d'une structure cyclique à cinq chaînons, trois chaînons du cycle à cinq chaînons présentant la structure -O-C(=O)-S-, les deux autres chaînons du cycle à cinq chaînons étant des atomes de C ; et
(b) contenant dans un deuxième composant au moins un composé durcisseur B qui est choisi parmi des composés qui présentent au moins un groupe fonctionnel qui est choisi parmi des groupes amine primaire et des groupes amine secondaire,
éventuellement dans le premier composant, dans le deuxième composant et/ou dans un composant supplémentaire de l'adhésif, au moins un composé C pouvant être contenu qui présente au moins un groupe fonctionnel réactif envers des groupes SH.

23. Produit collé, préparé selon le procédé selon l'une quelconque des revendications 1 à 21.

24. Composition durcissable d'adhésif à 2 composants, qui
(a) contient dans un premier composant au moins un composé A doté d'un poids moléculaire supérieur à 1 000 g/mole et comportant au moins un motif de cyclothiocarbonate doté d'une structure cyclique à cinq chaînons, trois chaînons du cycle à cinq chaînons présentant la structure -O-C(=O)-S-, les deux autres chaînons du cycle à cinq chaînons étant des atomes de C ; et
(b) contient dans un deuxième composant au moins un composé durcisseur B qui est choisi parmi des composés qui présentent au moins un groupe fonctionnel qui est choisi parmi des groupes amine primaire et des groupes amine secondaire, l'au moins un groupe fonctionnel pouvant être présent également sous forme masquée, à réactivité latente ; et
(c) éventuellement au moins un composé C étant contenu dans le premier composant, dans le deuxième composant et/ou dans un composant supplémentaire, lequel contient au moins un groupe fonctionnel réactif envers des groupes SH, de préférence au moins un groupe comportant une liaison éthyléniquement insaturée ou au moins un groupe époxy, dans laquelle dans le cas de composés comportant des groupes époxy, ceux-ci sont contenus dans le premier composant et/ou dans le composant supplémentaire de l'adhésif ;
le composé durcisseur B étant utilisé en une quantité telle que la quantité n_{B} de groupes fonctionnels du composé durcisseur B soit de 80 % en moles à 120 % en moles, par rapport à la quantité n_{A} de groupes cyclothiocarbonate du composé A ; et le composé C étant utilisé en une quantité telle que la quantité des doubles liaisons réactives avec des groupes SH n_{C3} soit de 0 à 110 % en moles, de préférence de 90 à 110 % en moles, par rapport à la quantité n_{A} de groupes cyclothiocarbonate ; et la composition d'adhésif présentant, immédiatement après le mélange des composants à l'état non encore durci, de préférence une viscosité nulle à 70 °C inférieure à 300 Pa s.

25. Composition d'adhésif durcissable à 2 composants, selon la revendication précédente, **caractérisée en ce que** la résistance au pelage de deux feuilles de poly(téréphtalate d'éthylène) collées l'une avec l'autre avec une couche d'adhésif de 3 µm et une pression de 3 bars après 24 heures est supérieure à 1,5 N/15 mm.
